# EUROPEAN PATENT APPLICATION

(11) **EP 1 980 856 A1**
(43) Date of publication of application: **15.10.2008**
(21) Application number: 08251398.7
(22) Date of filing: 11.04.2008
(51) Int. Cl.: G01N 33/574

(54) **Detecting prostate cancer**

(30) Priority: 12.04.2007 US 734763
(71) Applicant: Veridex, LLC, Warren, NJ 07059 (US)
(72) Inventor: Wang, Haiying, Bridgwater, NJ 08807 (US); Baden, Jonathan, Bridgewater, NJ, 08807 (US); Vener, Tatiana, Sterling, NJ 07908 (US); Chowdary, Dondapati, Princeton Junction, NJ 08876 (US); Mazumder, Abhijit, Basking Ridge, NJ 07970 (US)
(74) Representative: Goodfellow, Hugh Robin

(57) **Abstract**

Methods and kits for detecting prostate cancer in urine samples include detecting the methylation status of various genes.

SEQUENCE LISTING

<110>   VERIDEX LLC

<120>   Detecting Prostate Cancer

<130>   P050151EP

<150>   US11/734763
<151>   2007-04-12

<160>   25

<170>   SeqWin99, version 1.02

<210>   1
<211>   18
<212>   DNA
<213>   Artificial Sequence

<220>
<223>   PCR primer

<400>   1
tcggggattt tagggcgt                                                    18

<210>   2
<211>   21
<212>   DNA
<213>   Artificial Sequence

<220>
<223>   PCR primer

<400>   2
acgaaaacta cgacgacgaa a                                                21

<210>   3
<211>   47
<212>   DNA
<213>   Artificial Sequence

<220>
<223>   Probe

<220>
<221>   modified_base
<222>   1
<223>   fam-

<220>
<221>   modified_base
<222>   32
<223>   BHQ-HEG-

<400>   3
cgcacggcga actcccgccg acgtgcgtgt agcggtcgtc ggggttg                    47

<210>   4
<211>   22
<212>   DNA
<213>   Artificial Sequence

<220>
<223>   Probe

<400>   4
gccccaatac taaatcacga cg                                               22

<210> 5
<211> 24
<212> DNA
<213> Artificial Sequence

<220>
<223> PCR primer

<400> 5
gatataaggt tagggatagg atag 24

<210> 6
<211> 22
<212> DNA
<213> Artificial Sequence

<220>
<223> PCR primer

<400> 6
aaccaataaa acctactcct cc 22

<210> 7
<211> 52
<212> DNA
<213> Artificial Sequence

<220>
<223> Probe

<220>
<221> modified_base
<222> 1
<223> q670-

<220>
<221> modified_base
<222> 28
<223> -BHQ2-HEG-

<400> 7
ccgcgcatca ccaccccaca cgcgcgggga gtatataggt tggggaagtt tg 52

<210> 8
<211> 27
<212> DNA
<213> Artificial Sequence

<220>
<223> PCR primer

<400> 8
aacacacaat aacaaacaca aattcac 27

<210> 9
<211> 19
<212> DNA
<213> Artificial Sequence

<220>
<223> PCR primer

<400> 9
ccctataccc cactacgaa 19

<210> 10
<211> 25
<212> DNA
<213> Artificial Sequence

<220>
<223>   PCR primer

<400>   10
ggcgggttgt attaatatag ttata                                          25

<210>   11
<211>   44
<212>   DNA
<213>   Artificial sequence

<220>
<223>   Probe

<220>
<221>   modified_base
<222>   1
<223>   texas red -

<220>
<221>   modified_base
<222>   25
<223>   -BHQ2-HEG-

<400>   11
gccggcgggt tttcgacggg ccggccgaac caaaacgctc ccca                     44

<210>   12
<211>   25
<212>   DNA
<213>   Artificial Sequence

<220>
<223>   PCR primer

<400>   12
gtcggttacg tgcgtttata tttag                                          25

<210>   13
<211>   25
<212>   DNA
<213>   Artificial Sequence

<220>
<223>   PCR primer

<400>   13
ggaagtgagt tgtttagagg tagga                                          25

<210>   14
<211>   25
<212>   DNA
<213>   Artificial Sequence

<220>
<223>   PCR primer

<400>   14
tccaaactta ctcgaccaat ccaac                                          25

<210>   15
<211>   47
<212>   DNA
<213>   Artificial Sequence

<220>
<223>   Probe

<220>
<221>   modified_base
<222>   1
<223>   q570

<220>
<221>   modified_base
<222>   27
<223>   -BHQ2-HEG-

<400>   15
cggcgcccga cgatacccaa agcgccgaac gcgagcgatt cgagtag                47

<210>   16
<211>   24
<212>   DNA
<213>   Artificial Sequence

<220>
<223>   PCR primer

<400>   16
cttacaaaaa accttccgaa tacg                                         24

<210>  17
<211>  4260
<212>  DNA
<213>  Homo sapiens

<400>   17
aacaagagat caatatctag aataaatgga gatctgcaaa tcaacagaaa gtaggcagca    60
aagccaaaga aaatagccta aggcacagcc actaaaagga acgtgatcat gtcctttgca   120
gggacatggg tggagctgga agccgttagc ctcagcaaac tcacacagga acagaaaacc   180
agcgagaccg catggtctca cttataagtg ggagctgaac aatgagaaca catggtcaca   240
tggcggcgat caacacacac tggtgcctgt tgagcggggt gctggggagg gagagtacca   300
ggaagaatag ctaagggata ctgggcttaa tacctgggtg atgggatgat ctgtacagca   360
aaccatcatg gcgcacacac ctatgtaaca aacctgcaca tcctgcacat gtaccccaga   420
acttcaaata aaagttggac ggccaggcgt ggtggctcac gcctgtaatc ccagcacttt   480
gggaagccga ggcgtgcaga tcacctaagg tcaggagttc gagaccagcc cggccaacat   540
ggtgaaaccc cgtctctact aaaaatacaa aaatcagcca gatgtggcac gcacctataa   600
ttccacctac tcgggaggct gaagcagaat tgcttgaacc cgagaggcgg aggttgcagt   660
gagccgccga gatcgcgcca ctgcactcca gcctgggcca cagcgtgaga ctacgtcata   720
aaataaaata aaataacaca aaataaaata aaataaaata aaataaaata aaataataaa   780
ataaaataaa ataaaataaa ataaaataaa ataaagcaat ttcctttcct ctaagcggcc   840
tccacccctc tcccctgccc tgtgaagcgg gtgtgcaagc tccgggatcg cagcggtctt   900
agggaatttc cccccgcgat gtcccggcgc gccagttcgc tgcgcacact tcgctgcggt   960
cctcttcctg ctgtctgttt actccctagg ccccgctggg gacctgggaa agagggaaag  1020
gcttccccgg ccagctgcgc ggcgactccg gggactccag ggcgcccctc tgcggccgac  1080
gcccggggtg cagcggccgc cggggctggg gccggcggga gtccgcggga ccctccagaa  1140
gagcggccgg cgccgtgact cagcactggg gcggagcggg gcgggaccac ccttataagg  1200
ctcggaggcc gcgaggcctt cgctggagtt tcgccgccgc agtcttcgcc accagtgagt  1260
acgcgcggcc cgctccccgg ggatggggct cagagctccc agcatggggc caacccgcag  1320
catcaggccc gggctcccgg cagggctcct cgcccacctc gagacccggg acgggggcct  1380
aggggaccca ggacgtcccc agtgccgtta gcggctttca gggggcccgg agcgcctcgg  1440
ggagggatgg gaccccgggg gcggggaggg ggggcaggct gcgctcaccg cgccttggca  1500
tcctcccccg ggctccagca aacttttctt tgttcgctgc agtgccgccc tacaccgtgg  1560
tctatttccc agttcgaggt aggagcatgt gtctggcagg gaagggaggc aggggctggg  1620
gctgcagccc acagcccctc gcccacccgg agagatccga acccccttat ccctccgtcg  1680
tgtggctttt accccgggcc tccttcctgt tccccgcctc tcccgccatg cctgctcccc  1740
gccccagtgt tgtgtgaaat cttcggagga acctgtttac ctgttccctc cctgcactcc  1800
tgacccctcc ccgggttgct gcgaggcgga gtcggcccgg tccccacatc tcgtacttct  1860
ccctccccgc aggccgctgc gcggccctgc gcatgctgct ggcagatcag ggccagagct  1920
ggaaggagga ggtggtgacc gtggagacgt ggcaggaggg ctcactcaaa gcctcctgcg  1980
taagtgacca tgcccgggca aggggagggg gtgctgggcc ttagggggct gtgactagga  2040
tcgggggacg cccaagctca gtgcccctcc ctgagccatg cctcccccaa cagctatacg  2100
ggcagctccc caagttccag gacggagacc tcaccctgta ccagtccaat accatcctgc  2160
gtcacctggg ccgcaccctt ggtgagtctt gaacctccaa gtccagggca ggcatgggca  2220
agcctctgcc cccggagccc ttttgtttaa atcagctgcc ccgcagccct ctggagtgga  2280
ggaaactgag acccactgag gttacgtagt ttgcccaagg tcaagcctgg gtgcctgcaa  2340

tccttgccct gtgccaggct gcctcccagg tgtcaggtga gctctgagca cctgctgtgt 2400
ggcagtctct catccttcca cgcacatcct cttcccctcc tcccaggctg gggctcacag 2460
acagccccct ggttggccca tccccagtga ctgtgtgttg atcaggcgcc cagtcacgcg 2520
gcctgctccc ctccacccaa ccccagggct ctatgggaag gaccagcagg aggcagccct 2580
ggtggacatg gtgaatgacg gcgtggagga cctccgctgc aaatacatct ccctcatcta 2640
caccaactat gtgagcatct gcaccagggt tgggcactgg gggctgaaca aagaaagggg 2700
cttcttgtgc cctcaccccc cttacccctc aggtggcttg ggctgacccc ttcttgggtc 2760
agggtgcagg ggctgggtca gctctgggcc aggggcccag gggcctggga caagacacaa 2820
cctgcaccct tattgcctgg gacatcaacc agccaagtaa cgggtcatgg gggcgagtgc 2880
aaggacagag acctccagca actggtggtt tctgatctcc tggggtggcg agggcttcct 2940
ggagtagcca gaggtggagg aggatttgtc gccagtttct ggatggaggt gctggcactt 3000
ttagctgagg aaaatatgca gacacagagc acatttgggg acctgggacc agttcagcag 3060
aggcagcgtg tgtgcgcgtg cgtgtgcgtg tgtgtgcgtg tgtgtgtgta cgcttgcatt 3120
tgtgtcgggt gggtaaggag atagagatgg gcgggcagta ggcccaggtc ccgaaggcct 3180
tgaacccact ggtttggagt ctcctaaggg caatgggggc cattgagaag tctgaacagg 3240
gctgtgtctg aatgtgaggt ctagaaggat cctccagaga agccagctct aaagcttttg 3300
caatcatctg gtgagagaac ccagcaagga tggacaggca gaatggaata gagatgagtt 3360
ggcagctgaa gtggacagga tttggtacta gcctggttgt ggggagcaag cagaggagaa 3420
tctgggactc tggtgtctgg cctggggcag acgggggtgt ctcaggggct gggagggatg 3480
agagtaggat gatacatggt ggtgtctggc aggaggcggg caaggatgac tatgtgaagg 3540
cactgcccgg gcaactgaag ccttttgaga ccctgctgtc ccagaaccag ggaggcaaga 3600
ccttcattgt gggagaccag gtgagcatct ggccccatgc tgttccttcc tcgccaccct 3660
ctgcttccag atggacacag gtgtgagcca tttgtttagc aaagcagagc agacctaggg 3720
gatgggctta ggccctctgc ccccaattcc tccagcctgc tcccgctggc tgagtcccta 3780
gcccccctgc cctgcagatc tccttcgctg actacaacct gctggacttg ctgctgatcc 3840
atgaggtcct agcccctggc tgcctggatg cgttccccct gctctcagca tatgtggggc 3900
gcctcagtgc ccggcccaag ctcaaggcct tcctggcctc ccctgagtac gtgaacctcc 3960
ccatcaatgg caacgggaaa cagtgagggt tggggggact ctgagcggga ggcagagttt 4020
gccttccttt ctccaggacc aataaaattt ctaagagagc tactatgagc actgtgtttc 4080
ctgggacggg gcttaggggt tctcagcctc gaggtcggtg ggagggcaga gcagaggact 4140
agaaaacagc tcctccagca cagtcagtgg cttcctggag ccctcagcct ggctgtgttt 4200
actgaacctc acaaactaga agaggaagaa aaaaaaagag agagagaaac aaagagaaat 4260

<210> 18
<211> 866
<212> DNA
<213> Homo sapiens

<220>
<221> misc_feature
<222> 315
<223> n = a, c, g or t

<400> 18
acttatatat ctgacagttg atttgtcctc acctctaaat tggaatttaa gcatcacctg 60
gttcgattta atgcaatgta gaatttgcat taaaatacta cattaaagcc tcagatttgt 120
agtagctaac agcacttcta tgtatgtgtc agggactgct ctaaatactt catatatatt 180
aactcctcta ttctgtactt ctgttcccgt tttatacagc aggaaattga aacactgaga 240
ggttaagtaa ctaaagttac agagctagag tgacaggagt aaagcttcaa ctcaggcaac 300
ccagacgtcc agagntctga tctccactac taagctgcta gcatagcttt tctggtaact 360
atttttaatt caatataatt cgaatgatct atctaacaag tcatcactct gacaactcag 420
tgacttgtaa tgtaaaatta ttcattgtaa ttcacttaat attattgttt ctctgtgctg 480
caaaaatcat agcaatcgag atgtaattta ttactctccc tcccacctcc ggcatcttgt 540
gctaatcctt ctgccctgcg gacctccccc gactctttac tatgcgtgtc aactgccatc 600
aacttccttg cttgctgggg actggggccg tgagggcata cccccgaggg gtacggggct 660
agggctaggc aggctgtgcg gttgggcggg gccctgtgcc ccactgcgga gtgcgggtcg 720
ggaagcggag agagaagcag ctgtgtaatc cgctggatgc ggaccagggc gctccccatt 780
cccgtcggga gcccgccgat tggctgggtg tgggcgcacg tgaccgacat gtggctgtat 840
tggtgcagcc cgccagggtg tcactg 866

<210> 19
<211> 10386
<212> DNA
<213> Homo sapiens

<220>
<221> misc_feature
<222> 9531
<223> n = a, c, g or t

<400> 19
attgaggact cggaaatgag gtccaagggt agccaaggat ggctgcagct tcatatgatc 60
agttgttaaa gcaagttgag gcactgaaga tggagaactc aaatcttcga caagagctag 120
aagataattc caatcatctt acaaaactgg aaactgaggc atctaatatg aaggaagtac 180
ttaaacaact acaaggaagt attgaagatg aagctatggc ttcttctgga cagattgatt 240
tattagagcg tcttaaagag cttaacttag atagcagtaa tttccctgga gtaaaactgc 300
ggtcaaaaat gtccctccgt tcttatggaa gccgggaagg atctgtatca agccgttctg 360
gagagtgcag tcctgttcct atgggttcat ttccaagaag agggtttgta aatggaagca 420
gagaaagtac tggatattta gaagaacttg agaaagagag gtcattgctt cttgctgatc 480
ttgacaaaga agaaaaggaa aaagactggt attacgctca acttcagaat ctcactaaaa 540
gaatagatag tcttccttta actgaaaatt tttccttaca aacagatatg accagaaggc 600
aattggaata tgaagcaagg caaatcagag ttgcgatgga agaacaacta ggtacctgcc 660
aggatatgga aaaacgagca cagcgaagaa tagccagaat tcagcaaatc gaaaaggaca 720
tacttcgtat acgacagctt ttacagtccc aagcaacaga agcagagagg tcatctcaga 780
acaagcatga aaccggctca catgatgctg agcggcagaa tgaaggtcaa ggagtgggag 840
aaatcaacat ggcaacttct ggtaatggtc agggttcaac tacacgaatg gaccatgaaa 900
cagccagtgt tttgagttct agtagcacac actctgcacc tcgaaggctg acaagtcatc 960
tgggaaccaa ggtggaaatg gtgtattcat tgttgtcaat gcttggtact catgataagg 1020
atgatatgtc gcgaactttg ctagctatgt ctagctccca agacagctgt atatccatgc 1080
gacagtctgg atgtcttcct ctcctcatcc agcttttaca tggcaatgac aaagactctg 1140
tattgttggg aaattcccgg ggcagtaaag aggctcgggc cagggccagt gcagcactcc 1200
acaacatcat tcactcacag cctgatgaca agagaggcag gcgtgaaatc cgagtccttc 1260
atcttttgga acagatacgc gcttactgtg aaacctgttg ggagtggcag gaagctcatg 1320
aaccaggcat ggaccaggac aaaaatccaa tgccagctcc tgttgaacat cagatctgtc 1380
ctgctgtgtg tgttctaatg aaactttcat ttgatgaaga gcatagacat gcaatgaatg 1440
aactaggggg actacaggcc attgcagaat tattgcaagt ggactgtgaa atgtacgggc 1500
ttactaatga ccactacagt attacactaa gacgatatgc tggaatggct ttgacaaact 1560
tgacttttgg agatgtagcc aacaaggcta cgctatgctc tatgaaaggc tgcatgagag 1620
cacttgtggc ccaactaaaa tctgaaagtg aagacttaca gcaggttatt gcaagtgttt 1680
tgaggaattt gtcttggcga gcagatgtaa atagtaaaaa gacgttgcga gaagttggaa 1740
gtgtgaaagc attgatggaa tgtgctttag aagttaaaaa ggaatcaacc ctcaaaagcg 1800
tattgagtgc cttatggaat ttgtcagcac attgcactga gaataaagct gatatatgtg 1860
ctgtagatgg tgcacttgca tttttggttg gcactcttac ttaccggagc cagacaaaca 1920
ctttagccat tattgaaagt ggaggtggga tattacggaa tgtgtccagc ttgatagcta 1980
caaatgagga ccacaggcaa atcctaagag agaacaactg tctacaaact ttattacaac 2040
acttaaaatc tcatagtttg acaatagtca gtaatgcatg tggaactttg tggaatctct 2100
cagcaagaaa tcctaaagac caggaagcat tatgggacat gggggcagtt agcatgctca 2160
agaacctcat tcattcaaag cacaaaatga ttgctatggg aagtgctgca gctttaagga 2220
atctcatggc aaataggcct gcgaagtaca aggatgccaa tattatgtct cctggctcaa 2280
gcttgccatc tcttcatgtt aggaaacaaa aagccctaga agcagaatta gatgctcagc 2340
acttatcaga aacttttgac aatatagaca atttaagtcc caaggcatct catcgtagta 2400
agcagagaca caagcaaagt ctctatggtg attatgtttt tgacaccaat cgacatgatg 2460
ataataggtc agacaatttt aatactggca acatgactgt cctttcacca tatttgaata 2520
ctacagtgtt acccagctcc tcttcatcaa gaggaagctt agatagttct cgttctgaaa 2580
aagatagaag tttggagaga gaacgcggaa ttggtctagg caactaccat ccagcaacag 2640
aaaatccagg aacttcttca aagcgaggtt tgcagatctc caccactgca gcccagattg 2700
ccaaagtcat ggaagaagtg tcagccattc atacctctca ggaagacaga agttctgggt 2760
ctaccactga attacattgt gtgacagatg agagaaatgc acttagaaga agctctgctg 2820
cccatacaca ttcaaacact tacaatttca ctaagtcgga aaattcaaat aggacatgtt 2880
ctatgcctta tgccaaatta gaatacaaga gatcttcaaa tgatagttta aatagtgtca 2940
gtagtagtga tggttatggt aaaagaggtc aaatgaaacc ctcgattgaa tcctattctg 3000
aagatgatga aagtaagttt tgcagttatg gtcaataccc agccgaccta gcccataaaa 3060
tacatagtgc aaatcatatg gatgataatg atggagaact agatacacca ataaattata 3120
gtcttaaata ttcagatgag cagttgaact ctggaaggca aagtccttca cagaatgaaa 3180
gatgggcaag acccaaacac ataatagaag atgaaataaa acaaagtgag caaagacaat 3240
caaggaatca aagtacaact tatcctgttt atactgagag cactgatgat aaacacctca 3300
agttccaacc acattttgga cagcaggaat gtgtttctcc atacaggtca cggggagcca 3360
atggttcaga aacaaatcga gtgggttcta atcatggaat taatcaaaat gtaagccagt 3420
ctttgtgtca agaagatgac tatgaagatg ataagcctac caattatagt gaacgttact 3480
ctgaagaaga acagcatgaa gaagaagaga gaccaacaaa ttatagcata aaatataatg 3540
aagagaaacg tcatgtggat cagcctattg attatagttt aaaatatgcc acagatattc 3600
cttcatcaca gaaacagtca ttttcattct caaagagttc atctggacaa agcagtaaaa 3660
ccgaacatat gtcttcaagc agtgagaata cgtccacacc ttcatctaat gccaagaggc 3720
agaatcagct ccatccaagt tctgcacaga gtagaagtgg tcagcctcaa aaggctgcca 3780
cttgcaaagt ttcttctatt aaccaagaaa caatacagac ttattgtgta gaagatactc 3840
caatatgttt ttcaagatgt agttcattat catctttgtc atcagctgaa gatgaaatag 3900
gatgtaatca gacgacacag gaagcagatt ctgctaatac cctgcaaata gcagaaataa 3960
aagaaaagat tggaactagg tcagctgaag atcctgtgag cgaagttcca gcagtgtcac 4020

agcaccctag aaccaaatcc agcagactgc agggttctag tttatcttca gaatcagcca 4080
ggcacaaagc tgttgaattt tcttcaggag cgaaatctcc ctccaaaagt ggtgctcaga 4140
cacccaaaag tccacctgaa cactatgttc aggagacccc actcatgttt agcagatgta 4200
cttctgtcag ttcacttgat agttttgaga gtcgttcgat tgccagctcc gttcagagtg 4260
aaccatgcag tggaatggta agtggcatta taagccccag tgatcttcca gatagccctg 4320
gacaaaccat gccaccaagc agaagtaaaa cacctccacc acctcctcaa acagctcaaa 4380
ccaagcgaga agtacctaaa aataaagcac ctactgctga aaagagagag agtggaccta 4440
agcaagctgc agtaaatgct gcagttcaga gggtccaggt tcttccagat gctgatactt 4500
tattacattt tgccacggaa agtactccag atggattttc ttgttcatcc agcctgagtg 4560
ctctgagcct cgatgagcca tttatacaga aagatgtgga attaagaata atgcctccag 4620
ttcaggaaaa tgacaatggg aatgaaacag aatcagagca gcctaaagaa tcaaatgaaa 4680
accaagagaa agaggcagaa aaaactattg attctgaaaa ggacctatta gatgattcag 4740
atgatgatga tattgaaata ctagaagaat gtattatttc tgccatgcca acaaagtcat 4800
cacgtaaagc aaaaaagcca gcccagactg cttcaaaatt acctccacct gtggcaagga 4860
aaccaagtca gctgcctgtg tacaaacttc taccatcaca aaacaggttg caaccccaaa 4920
agcatgttag ttttacaccg ggggatgata tgccacgggt gtattgtgtt gaagggacac 4980
ctataaactt ttccacagct acatctctaa gtgatctaac aatcgaatcc cctccaaatg 5040
agttagctgc tggagaagga gttagaggag gagcacagtc aggtgaattt gaaaaacgag 5100
ataccattcc tacagaaggc agaagtacag atgaggctca aggaggaaaa acctcatctg 5160
taaccatacc tgaattggat gacaataaag cagaggaagg tgatattctt gcagaatgca 5220
ttaattctgc tatgcccaaa gggaaaagtc acaagccttt ccgtgtgaaa aagataatgg 5280
accaggtcca gcaagcatct gcgtcgtctt ctgcacccaa caaaaatcag ttagatggta 5340
agaaaaagaa accaacttca ccagtaaaac ctataccaca aaatactgaa tataggacac 5400
gtgtaagaaa aaatgcagac tcaaaaaata atttaaatgc tgagagagtt ttctcagaca 5460
acaaagattc aaagaaacag aatttgaaaa ataattccaa ggacttcaat gataagctcc 5520
caaataatga agatagagtc agaggaagtt ttgcttttga ttcacctcat cattacacgc 5580
ctattgaagg aactccttac tgtttttcac gaaatgattc tttgagttct ctagattttg 5640
atgatgatga tgttgacctt tccagggaaa aggctgaatt aagaaaggca aaagaaaata 5700
aggaatcaga ggctaaagtt accagccaca cagaactaac ctccaaccaa caatcagcta 5760
ataagacaca agctattgca aagcagccaa taaatcgagg tcagcctaaa cccatacttc 5820
agaaacaatc cacttttccc cagtcatcca aagacatacc agacagaggg gcagcaactg 5880
atgaaaagtt acagaatttt gctattgaaa atactccagt ttgcttttct cataattcct 5940
ctctgagttc tctcagtgac attgaccaag aaaacaacaa taaagaaaat gaacctatca 6000
aagagactga gccccctgac tcacagggag aaccaagtaa acctcaagca tcaggctatg 6060
ctcctaaatc atttcatgtt gaagataccc cagtttgttt ctcaagaaac agttctctca 6120
gttctcttag tattgactct gaagatgacc tgttgcagga atgtataagc tccgcaatgc 6180
caaaaaagaa aaagccttca agactcaagg gtgataatga aaaacatagt cccagaaata 6240
tgggtggcat attaggtgaa gatctgacac ttgatttgaa agatatacag agaccagatt 6300
cagaacatgg tctatcccct gattcagaaa attttgattg gaaagctatt caggaaggtg 6360
caaattccat agtaagtagt ttacatcaag ctgctgctgc tgcatgttta tctagacaag 6420
cttcgtctga ttcagattcc atcctttccc tgaaatcagg aatctctctg ggatcaccat 6480
ttcatcttac acctgatcaa gaagaaaaac cctttacaag taataaaggc ccacgaattc 6540
taaaaccagg ggagaaaagt acattggaaa ctaaaaagat agaatctgaa agtaaaggaa 6600
tcaaaggagg aaaaaaagtt tataaaagtt tgattactgg aaaagttcga tctaattcag 6660
aaatttcagg ccaaatgaaa cagccccttc aagcaaacat gccttcaatc tctcgaggca 6720
ggacaatgat tcatattcca ggagttcgaa atagctcctc aagtacaagt cctgtttcta 6780
aaaaaggccc accccttaag actccagcct ccaaaagccc tagtgaaggt caaacagcca 6840
ccacttctcc tagaggagcc aagccatctg tgaaatcaga attaagccct gttgccaggc 6900
agacatccca aataggtggg tcaagtaaag caccttctag atcaggatct agagattcga 6960
ccccttcaag acctgcccag caaccattaa gtagacctat acagtctcct ggccgaaact 7020
caatttcccc tggtagaaat ggaataagtc ctcctaacaa attatctcaa cttccaagga 7080
catcatcccc tagtactgct tcaactaagt cctcaggttc tggaaaaatg tcatatacat 7140
ctccaggtag acagatgagc caacagaacc ttaccaaaca aacaggttta tccaagaatg 7200
ccagtagtat tccaagaagt gagtctgcct ccaaaggact aaatcagatg aataatggta 7260
atggagccaa taaaaaggta gaactttcta gaatgtcttc aactaaatca agtggaagtg 7320
aatctgatag atcagaaaga cctgtattag tacgccagtc aactttcatc aaagaagctc 7380
caagcccaac cttaagaaga aaattggagg aatctgcttc atttgaatct ctttctccat 7440
catctagacc agcttctccc actaggtccc aggcacaaac tccagtttta agtccttccc 7500
ttcctgatat gtctctatcc acacattcgt ctgttcaggc tggtggatgg cgaaaactcc 7560
cacctaatct cagtcccact atagagtata atgatggaag accagcaaag cgccatgata 7620
ttgcacggtc tcattctgaa agtccttcta gacttccaat caataggtca ggaacctgga 7680
aacgtgagca cagcaaacat tcatcatccc ttcctcgagt aagcacttgg agaagaactg 7740
gaagttcatc ttcaattctt tctgcttcat cagaatccag tgaaaaagca aaaagtgagg 7800
atgaaaaaca tgtgaactct atttcaggaa ccaaacaaag taaagaaaac caagtatccg 7860
caaaaggaac atggagaaaa ataaaagaaa atgaattttc tcccacaaat agtacttctc 7920
agaccgtttc ctcaggtgct acaaatggtg ctgaatcaaa gactctaatt tatcaaatgg 7980
cacctgctgt ttctaaaaca gaggatgttt gggtgagaat tgaggactgt cccattaaca 8040
atcctagatc tggaagatct cccacaggta atactccccc ggtgattgac agtgtttcag 8100
aaaaggcaaa tccaaacatt aaagattcaa aagataatca ggcaaaacaa aatgtgggta 8160

atggcagtgt tcccatgcgt accgtgggtt tggaaaatcg cctgaactcc tttattcagg 8220
tggatgcccc tgaccaaaaa ggaactgaga taaaaccagg acaaaataat cctgtccctg 8280
tatcagagac taatgaaagt tctatagtgg aacgtacccc attcagttct agcagctcaa 8340
gcaaacacag ttcacctagt gggactgttg ctgccagagt gactcctttt aattacaacc 8400
caagccctag gaaaagcagc gcagatagca cttcagctcg gccatctcag atcccaactc 8460
cagtgaataa caacacaaag aagcgagatt ccaaaactga cagcacagaa tccagtggaa 8520
cccaaagtcc taagcgccat tctgggtctt accttgtgac atctgtttaa aagagaggaa 8580
gaatgaaact aagaaaattc tatgttaatt acaactgcta tatagacatt ttgtttcaaa 8640
tgaaacttta aaagactgaa aaattttgta aataggtttg attcttgtta gagggttttt 8700
gttctggaag ccatatttga tagtatactt tgtcttcact ggtcttattt tgggaggcac 8760
tcttgatggt taggaaaaaa atagtaaagc caagtatgtt tgtacagtat gttttacatg 8820
tatttaaagt agcatcccat cccaacttcc tttaattatt gcttgtctta aaataatgaa 8880
cactacagat agaaaatatg atatattgct gttatcaatc atttctagat tataaactga 8940
ctaaacttac atcagggaaa aattggtatt tatgcaaaaa aaaatgtttt tgtccttgtg 9000
agtccatcta acatcataat taatcatgtg gctgtgaaat tcacagtaat atggttcccg 9060
atgaacaagc tttacccagc ctgtttgctt tactgcatga atgaaactga tggttcaatt 9120
tcagaagtaa tgattaacag ttatgtggtc acatgatgtg catagagata gctacagtgt 9180
aataatttac actattttgt gctccaaaca aaacaaaaat ctgtgtaact gtaaaacatt 9240
gaatgaaact attttacctg aactagattt tatctgaaag taggtagaat ttttgctatg 9300
ctgtaatttg ttgtatattc tggtatttga ggtgagatgg ctgctctttt attaatgaga 9360
catgaattgt gtctcaacag aaactaaatg aacatttcag aataaattat tgctgtatgt 9420
aaactgttac tgaaattggt atttgtttga agggtcttgt ttcacatttg tattaataat 9480
tgtttaaaat gcctctttta aaagcttata taaatttttt ncttcagctt ctatgcatta 9540
agagtaaaat tcctcttact gtaataaaaa caattgaaga agactgttgc cacttaacca 9600
ttccatgcgt tggcacttat ctattcctga aattctttta tgtgattagc tcatcttgat 9660
ttttaacatt tttccactta aacttttttt tcttactcca ctggagctca gtaaaagtaa 9720
attcatgtaa tagcaatgca agcagcctag cacagactaa gcattgagca taataggccc 9780
acataatttc ctctttctta atattataga aattctgtac ttgaaattga ttcttagaca 9840
ttgcagtctc ttcgaggctt tacagtgtaa actgtcttgc cccttcatct tcttgttgca 9900
actgggtctg acatgaacac tttttatcac cctgtatgtt agggcaagat ctcagcagtg 9960
aagtataatc agcactttgc catgctcaga aaattcaaat cacatggaac tttagaggta 10020
gatttaatac gattaagata ttcagaagta tattttagaa tccctgcctg ttaaggaaac 10080
tttatttgtg gtaggtacag ttctggggta catgttaagt gtccccttat acagtggagg 10140
gaagtcttcc ttcctgaagg aaaataaact gacacttatt aactaagata atttacttaa 10200
tatatcttcc ctgatttgtt ttaaaagatc agagggtgac tgatgataca tgcatacata 10260
tttgttgaat aaatgaaaat ttatttttag tgataagatt catacactct gtatttgggg 10320
agagaaaacc tttttaagca tggtggggca ctcagatagg agtgaataca cctacctggt 10380
ggtcat 10386

<210> 20
<211> 2762
<212> DNA
<213> Homo sapiens

<400> 20
gtgacagaag tagtaggaag tgagctgttc agaggcagga gggtctattc tttgccaaag 60
gggggaccag aattccccat gcgagctgtt tgaggactgg gatgccgaga acgcgagcga 120
tccgagcagg gtttgtctgg gcaccgtcgg ggtaggatcc ggaacgcatt cggaaggctt 180
tttgcaagca tttacttgga aggagaactt gggatctttc tgggaacccc ccgccccggc 240
tggattggcc gagcaagcct ggaaaatgca attgaaacac agagcaccag ctctgaggaa 300
ctcgtcccaa gccccccatc tccacttcct ccccctcgag tgtacaaacc ctgcttcgtc 360
tgccaggaca aatcatcagg gtaccactat ggggtcagcg cctgtgaggg atgtaagggc 420
tttttccgca gaagtattca gaagaatatg atttacactt gtcaccgaga taagaactgt 480
gttattaata aagtcaccag gaatcgatgc caatactgtc gactccagaa gtgctttgaa 540
gtgggaatgt ccaaagaatc tgtcaggaat gacaggaaca agaaaaagaa ggagacttcg 600
aagcaagaat gcacagagag ctatgaaatg acagctgagt tggacgatct cacagagaag 660
atccgaaaag ctcaccagga aactttccct tcactctgcc agctgggtaa atacaccacg 720
aattccagtg ctgaccatcg agtccgactg gacctgggcc tctgggacaa attcagtgaa 780
ctggccacca agtgcattat taagatcgtg gagtttgcta aacgtctgcc tggtttcact 840
ggcttgacca tcgcagacca aattaccctg ctgaaggccg cctgcctgga catcctgatt 900
cttagaattt gcaccaggta taccccagaa caagacacca tgactttctc agacggcctt 960
accctaaatc gaactcagat gcacaatgct ggatttggtc ctctgactga ccttgtgttc 1020
acctttgcca accagctcct gcctttggaa atggatgaca cagaaacagg ccttctcagt 1080
gccatctgct taatctgtgg agaccgccag gaccttgagg aaccgacaaa agtagataag 1140
ctacaagaac cattgctgga agcactaaaa atttatatca gaaaaagacg acccagcaag 1200
cctcacatgt ttccaaagat cttaatgaaa atcacagatc tccgtagcat cagtgctaaa 1260
ggtgcagagc gtgtaattac cttgaaaatg gaaattcctg gatcaatgcc acctctcatt 1320
caagaaatgc tggagaattc tgaaggacat gaacccttga ccccaagttc aagtgggaac 1380
acagcagagc acagtcctag catctcaccc agctcagtgg aaaacagtgg ggtcagtcag 1440

tcaccactcg tgcaataaga cattttctag ctacttcaaa cattccccag taccttcagt 1500
tccaggattt aaaatgcaag aaaaaacatt tttactgctg cttagttttt ggactgaaaa 1560
gatattaaaa ctcaagaagg accaagaagt tttcatatgt atcaatatat atactcctca 1620
ctgtgtaact tacctagaaa tacaaacttt tccaatttta aaaaatcagc catttcatgc 1680
aaccagaaac tagttaaaag cttctatttt cctctttgaa cactcaagat gcatggcaaa 1740
gacccagtca aaatgattta cccctggtta agtttctgaa gactttgtac atacagaagt 1800
atggctctgt tctttctata ctgtatgttt ggtgctttcc ttttgtcttg catactcaaa 1860
ataaccatga caccaaggtt atgaaataga ctactgtaca cgtctaccta ggttcaaaaa 1920
gataactgtc ttgctttcat ggaatagtca agacatcaag gtaaggaaac aggactattg 1980
acaggactat tgtacagtat gacaagataa ggctgaagat attctacttt agttagtatg 2040
gaagcttgtc tttgctcttt ctgatgctct caaactgcat cttttatttc atgttgccca 2100
gtaaaagtat acaaattccc tgcactagca gaagagaatt ctgtatcagt gtaactgcca 2160
gttcagttaa tcaaatgtca tttgttcaat tgttaatgtc actttaaatt aaaagtggtt 2220
tattacttgt ttaatgacat aactacacag ttagttaaaa aaaatttttt tacagtaatg 2280
atagcctcca aggcagaaac acttttcagt gttaagtttt tgtttacttg ttcacaagcc 2340
attagggaaa tttcatggga taattagcag gctggtctac cactggacca tgtaactcta 2400
gtgtccttcc tgattcatgc ctgatattgg gatttttttc cagcccttct tgatgccaag 2460
ggctaattat attacatccc aaagaaacag gcatagaatc tgcctccttt gaccttgttc 2520
aatcactatg aagcagagtg aaagctgtgg tagagtggtt aacagataca agtgtcagtt 2580
tcttagttct catttaagca ctactggaat tttttttttt gatatattag caagtctgtg 2640
atgtactttc actggctctg tttgtacatt gagattgttt gtttaacaat gctttctatg 2700
ttcatatact gtttaccttt ttccatggac tctcctggca aagaataaaa tatatttatt 2760
tt 2762

<210> 21
<211> 8670
<212> DNA
<213> Homo sapiens

<400> 21
gagctcaaga gttcaagacc cgtctgggca agatggcaaa actccatcac cacaaaagat 60
gcaaaaagat gcgcacagtg gcgcacacct atagccccag ttactgagga ggttaatgtg 120
ggaggatcac atgaggctgc agtgagctgt gatggtgcca ctgtactcca gccttggcga 180
cagtgagtct atgtctcaaa taagtaagta aacaaaaatt aaaaagaatc cagtccacag 240
ggcatttgaa ggcaagagga aaagatgcca gaatcagaga tggggagaag atgggcttca 300
cgcacctgct gaggttgaga aatgagacag ataggctgag tgtggggtgg agagaggatg 360
ggcagagaga ctgaggctgg tctgaatgga aatgaaatgt tagggctctc agggttatcg 420
gggaataatt ggagcttcta ggaaaggttt aacgttgtga ccacctgtgt gcgtcatgcc 480
tccccacccc ttactaattg tgtgaatttg gcagactttg agtctcagtg ttctcctctg 540
tgaagtgggg tcatcttatt ccaactcctg ggattgttgt gtgaattaaa tggggtaatg 600
tacggagagc acctgacgca cagcgagtgc ttcaaaattt cagtctgcac cccccagcaa 660
aggatatgca cacgcccatt gtgagtgaca aatccaggat gacctgaacc caatgtgata 720
acgtgggtcc tcgcatgctg gtcatgctgc cgggagacac ttatggatcc aattagtaca 780
acaggggaaa taaattattt aatgcatttt gctaagacag aatacctcag aacttatttt 840
gtggggtggg gcataataaa gggggtcctt ctgctgaaaa cgtttaagct caggttcgtg 900
gcaccactca accaaggtcg acagtcacac agtaagccag aggcaatgtc aggacttaaa 960
ctaaacctgt ggcccccaca atgaggccat ttctctttcc cctgaacggc ctggggaaag 1020
ggggtgggtg ggcagaactt ggcagtggcc aatccctcac ttctgtcccc tggttttctc 1080
ctgcccttat ctctaggctt gcattgattg attgattgag acagggtctt gctctgtcgt 1140
ccaggctgga gtgcagtggc acgatcatgg ctcactgcag cctcaaactc ctaggctcaa 1200
gtggtctttc cgcctcctat ctcccgagta cccatatccc taggctttta aaatggcttc 1260
caggtatctg gctgccgtct cagacatcca cctgggcttc tgggcaggga ctgtccggga 1320
aacctcatct atgtgaagca ggtgtgggtg taggaaggcc gcttggaaat gaatcagcac 1380
tgtctcctgt ttgagtcgta agcagggcgc cagagggtct ggcggacaag aaagggagga 1440
tgacaggagg ccggcactgc aatgacacgc cttagccacc agagggcacg aagcagctgg 1500
gcaaaatccc gcggggcccc tggtggaaaa tttctggcac ctggagcccg gagatggggt 1560
ggacggaatg tgaggaccca gcttcctgag gctgggccgg ggcagagtca ctgctttgga 1620
tgtccgcagg gcctgcttgt gtcttgacta ctctgccttt gtagacagct ggagaatgtg 1680
agagtgggat tgggatcgga ctctagggcc attccgtaca actctcctgc cctgccgtgg 1740
gggagggagt tgcccaaggt tacgcagcaa gttagtggca aatgaatacg attatcacca 1800
gtctcaggta tatggccatt tgatgggcgc agtcgcagcc tcagttcctg agacagagac 1860
acctgattaa ggacaggcct tcaggagctg accctagtga cccgcggctc tgctgctgtc 1920
tctgtttttc tccctggctt ttccatctga ctgactcttt gtcttcttcg tctgcctgcc 1980
tgtctccgtc tctgcccgct ggggggtttg ctcaactccc tcactgggtc ctgggagccg 2040
cagtttcctg ctgtcactcc tcagggattt gtagctctct gaagctcttt tccgacccgt 2100
tgtctcggtt ccactcttgg gatccagagg agaggtgatt atttcgtagc atagtcagtg 2160
gtgtgatttc acggggtgag aaggactccc ttgctcctaa gcactcctcc agtgacccct 2220
gttgccatgt ggtagccgta agcactggtt ggcacctggt gtgggcgaga cccttacctc 2280
atgcagaaat gagtaagact ggtgagctca ctatgtgggg tgaggctgag agaaaacaag 2340

tacacaggtg attcagtcaa aatcagaatt ctctaagtac acacgaaaag ggcaaaaggg 2400
gcgctttgta caggacagaa caggtagaca ctgaatccgg ttgggccctg ggaaggctcc 2460
ctgcagtggc ctttgaaggg ggggttggat ttcagcagga tagagggcat gggcatgtgt 2520
gggcacgttc tgaacagagg ggtcagcgca agccgagggt cttggccaca ctagttgcat 2580
gtgccggtgt gtttaaggga cacgcagcag caggccgagt ctggagcgcc tcactgccag 2640
gctttttaaa aatttttaat tttaatttaa ttttatttta tttttacttt aagttctggc 2700
atacatgtgc agaatgtggt ttgttacata ggtatacatg tgccatggtg gtttgctgca 2760
cctatcaacc catcatctag gttttaagcc ccgcatgcat caggtattag tcctaatgct 2820
ctccctcccc ttgcccccat ccttctcccc gcaactgccc acaggccctg gtatgtggtg 2880
ttcccttccc tgtgtccata tgttctcatt gttcaactcc cacttatgag tgagaacata 2940
ccgcctggct ttaagggaca gccatgggga tgcactgcag tttctgagca gggaaggccc 3000
tgtggaggcc cttagttaaa aggaaagaat ggctgtgaaa atcgatgcat tgcgctccct 3060
tgtccctcac cctcagtgtg aagggttttt attccgagtt ctacttgaag taggcctcga 3120
tgggaagaca agtagcatga ggggttcaag tactgagggg agcaagggac actcggtggc 3180
tgtgccaagg tgtagaagag gacactgggg gccccaagac ctgacttcat gtacactgct 3240
caggctggcc cccaagtcac acggtgaccg ctaggaaggg accagcctgt tctcagtctg 3300
atcctacagc catgtcatta tccaaagctc ctcctggcag ggcctgtttg gggtctctgt 3360
gccagtgctt tccctgccag gctgggctgg ggcttccacc tactgctctg ggactgctgc 3420
tgccctggcc ctgggggagg agggtgtgcc gctgagtcac tgcctgggca tctgggcctg 3480
gaacctcggg tgagtcactt agggctgagg tagaggggct gggggagggg aagaagctac 3540
tcgacagctg gagcagggag gggagctggg gccacaggaa gggcggtgcc ctgatgccca 3600
gacgggccgg gatagacaaa gggccaagga ggaaggggcc ctgggagggg gcagccctcc 3660
cttgggctgg ggtctgaatg gcacagtgtt tgcctttctc cgggtctggg gaggacatgt 3720
gtgtgggggg cagtgagaga gggctgtggc tgagggctgt gcttcaggcc tggattctgg 3780
cttgggaagc tgtccagctg gtgttttcag ccttgggtag ggatgtaccc ctacccaccc 3840
acccagccct caagctggag aagaggaggc caaagttttc ctgttcagcc tttaactact 3900
cgggacttcc ttatgctccc cacagactgt ggcccagccc aactgcggct gtgtgtagag 3960
caaccccatt tctcactgct tccccatcct tccagacacc ttcctacaca gagggacctt 4020
cccaggtatt tctaagcaca cttagttacc tcattacctc attaagaggt attctggtgc 4080
tggccattaa aagtcactcc acttcatcca tgccctgaag tcagtcctgt ccttctcctc 4140
ctgatgtccc ccagctgcct cctctggccc ccagcttcct aaggtggccc caggttgctt 4200
ctctctcaca cacacgggcg catgtatgta cacgagcact ggaccatgaa gtctcagcgt 4260
gtgctcacag cctctcacac aggagtgggc tgtgactcac aggcatgtca tgagaatgag 4320
gcctggcacc agtctccagg ccccagagca ggggttgcct cccctcaccc cggtccagga 4380
tgcccagtcc ccacgacacc tcccacttcc cactgtggcc tgggtgggct caggggctgc 4440
ccttgacctg gcctagagcc ctcccccagc tggtggtgga gctggcactc tctgggaggg 4500
agggggctgg gagggaatga gtgggaatgg caagaggcca gggtttggtg ggatcaggtt 4560
gaggcaggtt tggtttcctt aaaatgccaa gttgggggcc agtggggccc acatataaat 4620
cctcaccctg ggagcctggc tgccttgctc tccttcctgg gtctgtctct gccacctggt 4680
ctggtgagta cctctgtcct gctgagggca gggtggggag gatccccgtg ggtctctgtc 4740
tttgtctcca cagttctctc attccagctt ccctggtggg atcaacctgg gcctctctgg 4800
gccttccccc ttggaagaac tctctgtgaa gtgctgaagt gttgactgaa gggttttttt 4860
tttttttttt tttttttgag atggagtctc gctctgtcgc ccaggctgga gtacagtggt 4920
gtgatctcag ctcactgcaa actccccctc ccaggttcac gccatttccc tgcctcagcc 4980
tcccgagtag ctgggactgc aggcgcccac caccatgccc ggctaatttt tttgtatttt 5040
tagtagagat ggggtttcac catgttagcc aggatggtct cgatctcctg atctcgtgat 5100
ccacccatct cggcctccca aagtgctggg attacaggag taagccaccg cgcccggccg 5160
actgaagggt ttttctccag gttcctctgt gaggtctcag tgcaggggtt gctctgaggc 5220
cctcccctgg atatctcagt ctaggggccc ttctttgggg gtctaggcct aggagcagga 5280
ggtgtgcatg tgggcgttgc tgcaaaaaga atcctgagat tttttttttt tttttttttt 5340
ttgcaaagtc ctggattcta gcaggactaa ggtgcaagag gcaggggtct caagactctg 5400
cctgggtcat ggccccaagc agcaaagctc tgccccctgc ctcggtgaag gcagggctgg 5460
catgatgggc ccagggcatg ccctgcctct ggcatagctc ctctggcctc accctgaaac 5520
ctgcctaacc tttccaggct ggtctgagta ttctcagagg ccttgccgct gaggtctgtc 5580
ccatcctgat cccaaggcaa tgaacatttc atatctttaa ttctaattcc aacaggatcc 5640
ttcctggtgg agagaatgtt aagttgcccc caccctatcc atgcccctgt ctgcctagag 5700
gctcaggggc cttcagggtg aggggagaca cattccccac cctctgggag ctcctagtct 5760
gagagaggaa acactcctgc ccaagggagc ttccagttag atggcagaga gagatgcctc 5820
tggcttcagg agtcccgagt ctaaggaggg aaacgactcc ttcagggagc ttcctgctcc 5880
taggctgtag ccatggctcc tgccagactg cacaggagcc cccatctgcc agccggtgca 5940
tgtggccctg ctccccagag cctgcgcaga tgccatcaaa atgggactct ggtcaccctg 6000
tcatttccct tctggcagac actaaaatgg ggagccctgc cctcaggggg gtgtcccaag 6060
tgccatcaga ggaggcttgg tgactcccag acacaaggga agctttagcg tctgccctca 6120
gggtgagatg gaggtatccc tccggcctca gggaaccaca gtctgagggg agatgcagcc 6180
cctgccttcc cattcagaga ggggttttgt gaggtggctt gggggcatag ggcagaagtg 6240
gatcctacag gctgagctaa ggccccaaga gcctcagcag tgtacccatc acctggcacc 6300
tctgcagcca cagatccatg atgtgcagtt ctctggagca ggcgctggct gtgctggtca 6360
ctaccttcca caagtactcc tgccaagagg gcgacaagtt caagctgagt aagggggaaa 6420
tgaaggaact tctgcacaag gagctgccca gctttgtggg ggtgagtggc acaggcctgt 6480

gggggaggtc ctggtgtgag tgtgggggtg caggttaaat ctctccccca gttccgggtg 6540
cctgtcgatg caggtgccag ggtggggccc agcccctccc cactttagct tcatggctcc 6600
actggagtgg aaatgaggcc cgagtgggag tgcttaatta atggctgttt cctgcaacat 6660
tccagagaac catgtgctgt gagggccttc cgagtccatc tgtttaatcc tgtcattgga 6720
acttgagaaa ccagagccca gaagggaaaa gtgattgtcc caagatcaca cagcactggc 6780
acgttctctc tctctctttt cttttctttt tttttttttg agatggagtt tccctcttgt 6840
tgcccaggct ggagtgcaat ggcacgatct cggctcactg caacctctgc ctccaggggt 6900
caagcaattc tcctgtctca gcctcctgag tagctgggac tacaggcgca tcccactacg 6960
cccagctaat ttttgtattt ttagtagaga cagggtttca ccatattggc caggctggtc 7020
tcgaactcct gacctcgtga tctacctgcc tcggcttccc aaagtgattt ttgtattttt 7080
agtagagacg gggtttcatc atattggtca ggctggtctc gaactcctga cctcaggtga 7140
tctgccctcc tcggcctctg aaagtgctgg gcttacaggc gtgagcaccg tgcccggact 7200
cctttttttt tttttttttt ttgtggtggg gggacaagat ctcactctgt cacccaggct 7260
ggatcatagc tcactgtaat ctcgaactcc tgggctcaag caatcctccc aagtagttgg 7320
aactacagga gtattgtcac catgcctggc caatttttat tttttgtaga gatggagtct 7380
tgctatgttg tccaggctgg gcttgaactc ctgggttcaa gcaatcctcc cacctcggcc 7440
tcccaaagta ttggaattac agatgtgagc cactgtgctt gacctctttc cattttttata 7500
tgccaaacta agaaagtatg ttagggatag aaaagccctg ctcagatata tagtctggga 7560
cattttgtgg agaaatgcat cgaccttcaa tttgtccctc accctcccta tactgactca 7620
ttggtgattc ccaaagttag gtgtcaggct ttgaacacat gaggcaggtc cttctttcct 7680
tggtttaatt ttgtttttgt ggctggttaa atttttctaa ttatttcggc tagtattaaa 7740
aaagtgtttt tcagctgggt gcagtggcct atgcctgtaa tccccacagt gtgggaggct 7800
aaggcaggag gatctcttaa gcccaggagt tcgaccagcc tgggcaacat agcaagactc 7860
catctctaca aaaataaaaa taaaaattgg ccaggcatgg tggcatacgc ttgtagtccc 7920
agctacttgg gaggctaaag gtgggaggat tgctggagcc caggaggttg aggctgcagt 7980
gagttgtgat tgtgccactg cactccaacc tgggctaaca gagcaagacc ttgtcttaaa 8040
aaataaaaag tgttcttttc tgaatctacc tggctggtgt tggggagcag caacttcggt 8100
ttcctcatca gcagaatggg gtgatgatac ctacctcgct gggctcctgt gggattcgag 8160
ctgatgcatg ctcagaggag catccagtgt cctccctgtg tccaggagga gggcacactg 8220
gagatgctca ccaatgagta tctgtctctc tccttactca ctgggccctc ttggtagctc 8280
ccagagcctc ctgcccacct tatacccagc tgcccagtgg ggagggagag ctggaaccaa 8340
cctgaatgtg tgagggtctg ggtgtttggt ggagctgggg ttggggctgg cttggtgatg 8400
agtgtatttc ctgtcacttt caggagaaag tggatgagga ggggctgaag aagctgatgg 8460
gcagcctgga tgagaacagt gaccagcagg tggacttcca ggagtatgct gttttcctgg 8520
cactcatcac tgtcatgtgc aatgacttct tccagggctg cccagaccga ccctgaagca 8580
gaactcttga cttcctgcca tggatctctt gggcccagga ctgttgatgc ctttgagttt 8640
tgtattcaat aaactttttt tgtctgttga 8670

<210> 22
<211> 2011
<212> DNA
<213> Homo sapiens

<400> 22
gagctctgtc tcttggccag ctgaatggag gcccagcggc aacacaggtc ctgcctgggg 60
atcaggtctg ctctgcaccc caccttgctg cctggagccg cccacctgac aacctctcat 120
ccctgctctg tagatccggt cccatcccca ctgcccaccc caccccccca gcactccacc 180
cagttcaacg ttccacgaac ccccagaacc agccctcatc aacaggcagc aagaagggcc 240
ccccgcccat cgccccacaa cgccagccgg gtgaactgta gcgttggcag gtcctgaggc 300
agctgaaaga tacaaggcca gggacaggac agtcccatcc ccaggaggca gggagtatac 360
aggctgggga agtttgccct tgcgtggggt ggtgatggag gaggctcagc aagtcttctg 420
gactgtgaac ctgtgtctgc cactgtgtgc tgggtggtgg tcatctttcc caccaggctg 480
tggcctctgc aaccttcaag ggaggagcag gtcccattgg ctgagcacag ccttgtacgt 540
gaactgaaca agcagcctcc ttcctggcca caggttccat gtccttatat ggactcatct 600
ttgcctattg cgacacacac tcaatgaaca cctactacgc gctgcaaaga gccccgcagg 660
cctgaggtgc ccccacctca ccactcttcc tatttttgtg taaaaatcca gcttcttgtc 720
accacctcca aggaggggga ggaggaggaa ggcaggttcc tctaggctga gccgaatgcc 780
cctctgtggt cccacgccac tgatcgctgc atgcccacca cctgggtaca cacagtctgt 840
gattcccgga gcagaacgga ccctgcccac ccggtcttgt gtgctactca gtggacagac 900
ccaaggcaag aaagggtgac aaggacaggg tcttcccagg ctggctttga gttcctagca 960
ccgccccgcc cccaatcctc tgtggcacat ggagtcttgg tccccagagt cccccagcgg 1020
cctccagatg gtctgggagg gcagttcagc tgtggctgcg catagcagac atacaacgga 1080
cggtgggccc agacccaggc tgtgtagacc cagccccccc gccccgcagt gcctaggtca 1140
cccactaacg ccccaggcct ggtcttggct gggcgtgact gttaccctca aaagcaggca 1200
gctccagggt aaaaggtgcc ctgccctgta gagcccactt ccttcccagg gctgcggctg 1260
ggtaggtttg tagccttcat cacgggccac ctccagccac tggaccgctg gcccctgccc 1320
tgtcctgggg agtgtggtcc tgcgactcta atggccgcaa gccacctgac tcccccaaca 1380
ccacactcta cctctcaagc ccaggtctct ccctagtgac ccacccagca catttagcta 1440
gctgagcccc acagccagag gtcctcaggc cctgctttca gggcagttgc tctgaagtcg 1500

gcaaggggga gtgactgcct ggccactcca tgccctccaa gagctccttc tgcaggagcg 1560
tacagaaccc agggccctgg cacccgtgca gaccctggcc caccccacct gggcgctcag 1620
tgcccaagag atgtccacac ctaggatgtc ccgcggtggg tgggggcccc gagagacggg 1680
caggccgggg gcaggcctgg ccatgcgggg ccgaaccggg cactgcccag cgtggggcgc 1740
gggggccacg gcgcgcgccc ccagcccccg ggcccagcac cccaaggcgg ccaacgccaa 1800
aactctccct cctcctcttc ctcaatctcg ctctcgctct tttttttttt cgcaaaagga 1860
ggggagaggg ggtaaaaaaa tgctgcactg tcggcgaagc cggtgagtga gcggcgcggg 1920
gccaatcgcg tgcgccgttc cgaaagttgc cttttatggc tcgagcggcc gcggcggcgc 1980
cctataaaac ccagcggcgc gacgcgccac c 2011

<210> 23
<211> 1792
<212> DNA
<213> Homo sapiens

<400> 23
   cgcgtccgcc ccgcgagcac agagcctcgc ctttgccgat ccgccgcccg tccacacccg 60
   ccgccagctc accatggatg atgatatcgc cgcgctcgtc gtcgacaacg gctccggcat 120
   gtgcaaggcc ggcttcgcgg gcgacgatgc cccccgggcc gtcttcccct ccatcgtggg 180
   gcgccccagg caccagggcg tgatggtggg catgggtcag aaggattcct atgtgggcga 240
   cgaggcccag agcaagagag gcatcctcac cctgaagtac cccatcgagc acggcatcgt 300
   caccaactgg gacgacatgg agaaaatctg gcaccacacc ttctacaatg agctgcgtgt 360
   ggctcccgag gagcaccccg tgctgctgac cgaggccccc ctgaacccca aggccaaccg 420
   cgagaagatg acccagatca tgtttgagac cttcaacacc ccagccatgt acgttgctat 480
   ccaggctgtg ctatccctgt acgcctctgg ccgtaccact ggcatcgtga tggactccgg 540
   tgacggggtc acccacactg tgcccatcta cgaggggtat gccctccccc atgccatcct 600
   gcgtctggac ctggctggcc gggacctgac tgactacctc atgaagatcc tcaccgagcg 660
   cggctacagc ttcaccacca cggccgagcg ggaaatcgtg cgtgacatta aggagaagct 720
   gtgctacgtc gccctggact tcgagcaaga gatggccacg gctgcttcca gctcctccct 780
   ggagaagagc tacgagctgc ctgacggcca ggtcatcacc attggcaatg agcggttccg 840
   ctgccctgag gcactcttcc agccttcctt cctgggcatg gagtcctgtg gcatccacga 900
   aactaccttc aactccatca tgaagtgtga cgtggacatc cgcaaagacc tgtacgccaa 960
   cacagtgctg tctggcggca ccaccatgta ccctggcatt gccgacagga tgcagaagga 1020
   gatcactgcc ctggcaccca gcacaatgaa gatcaagatc attgctcctc ctgagcgcaa 1080
   gtactccgtg tggatcggcg gctccatcct ggcctcgctg tccaccttcc agcagatgtg 1140
   gatcagcaag caggagtatg acgagtccgg cccctccatc gtccaccgca aatgcttcta 1200
   ggcggactat gacttagttg cgttacaccc tttcttgaca aaacctaact tgcgcagaaa 1260
   acaagatgag attggcatgg ctttatttgt tttttttgtt ttgttttggt tttttttttt 1320
   tttttggctt gactcaggat ttaaaaactg gaacggtgaa ggtgacagca gtcggttgga 1380
   gcgagcatcc cccaaagttc acaatgtggc cgaggacttt gattgcacat tgttgttttt 1440
   ttaatagtca ttccaaatat gagatgcatt gttacaggaa gtcccttgcc atcctaaaag 1500
   ccaccccact tctctctaag gagaatggcc cagtcctctc ccaagtccac acaggggagg 1560
   tgatagcatt gctttcgtgt aaattatgta atgcaaaatt tttttaatct tcgccttaat 1620
   acttttttat tttgttttat tttgaatgat gagccttcgt gccccccctt cccccttttt 1680
   gtcccccaac ttgagatgta tgaaggcttt tggtctccct gggagtgggt ggaggcagcc 1740
   agggcttacc tgtacactga cttgagacca gttgaataaa agtgcacacc tt 1792

<210> 24
<211> 7273
<212> DNA
<213> Homo sapiens

<400> 24
ggtacccagg ctggagtgca ctggtgtgat catagctcac taacctcgaa ctcctgggct 60
taggcaatcc tcttgccttg gcctcccaaa gtgccaggat tacaggcatg agccaccaca 120
gtggagctct caattctgat actaataatt tgtgtcttct cttttttttcc ttagcctgac 180
tagagtaatt aactttatgt cttttaaaag aaccaccttt ttggttttac ccattttctt 240
ttttgatttt ctgtttttga tttgattgat atctactcta atttttttatt atttcttttc 300
ctctgcttac tttgaattta attacttttc tttttttgtag tctcctaaaa tagaagctta 360
tattattgat tttagatctt tcttcttttc tattacagca ctcaatgcta taaatttccc 420
tctaagcatt gctttcactg catcctacaa tatttcaact ctattgttat ttagctcaaa 480
agaggttctt aatttctatt gggatttctc tttgacccat gtgttattca gaagtgttcc 540
gtgtgatctc caaatatttg ggagtttttc agctatcttt ctattaatca tttcttgttt 600
aattctattg tggcctgaga gcatatattg tatgatttat attcttgtaa atgtgttaag 660
gtgtgtctta tggtgcagaa tgcggtttat cttgctatat gttccttaga gaataatgta 720
tgttctgctg ttattggata aagtagtcta tagatgtcag ttacatctcg ttgattaatg 780
gtgctgttga gttcagctat gtcctaaatg attttctgtc tgctgtatct gtctatttct 840
gacacaaggc tgttgaagtc tccaaccata ataatgaatt aatctatttt tctttgcagt 900
tttatcaatt ttgtcttata tatattgatg ctccattgtt tggcacatac acattaagaa 960

ttgttatgtc ttcttggaga atttaccttt ccataacatg taacatttcc ctttattcct 1020
gataattttt cttgctcaaa agtttgccct gttggaaatt accagaacta ctctggcttt 1080
atttgattag tgttagcatg ctctctcttt ctctattctt acacttttaa tgtatacttg 1140
actttgtatt taaagtgggg ttcttataga aaacatatac ttggtagggt gggaagtaaa 1200
ataaaaagaa atacttgggt attggtttga tccactctaa caatctctat gttttaattg 1260
atgtatttag accattgata cttatttttt tatcctcatc cctgtgatta cccagagagc 1320
tgcttaaatt gattattgat atagacaaat taataattaa tatctaccgt ttgttactgt 1380
tttctatttt tcattgccct tactttctgc tcctattttt tgctcctttt tctgttaatt 1440
taggttttga gttattttat atcattctat tttctctccc ttctcagcat atgaattatc 1500
tttcttttttg acttttttag tggctgccct gaaggttgca atgtacattt acaaccagtc 1560
ccaatctcct ttcaaaaaac acaatactgt ttcatggcta gtgcaagtac ctaataataa 1620
gaagtcactc ctaatttctt tctctcattc tttgtatctt tactgttatt catttcactt 1680
gtacataagc tgtaatcttt caatacatta ttgctattat tatttcaaaa catgttatct 1740
attatatcta tttaaaataa gaaaaatagg ccaggtgcag tggcttactc atgtaatccc 1800
agcactttgg gagaccgatg gattgctaga gctcaggaat tcgagaccag cctgggcaac 1860
atagtgaaac cctgtctcta ctaaaaatac aaaaaaaaaa attgctgggc atggtggcat 1920
gggcctgtgg tcacagctac tcgggaggct gaggtgagag gattgcttga gcctgggagg 1980
cagaggttgc agtgaaccaa aatcaagcta ctgcactcca gcctaagtga cagagtgaga 2040
ccctgtctca aaaaaaaaat gaaagaatta tttttattta tcttcactta tttcttctct 2100
aatgctcttt gtttctttag tatgtagatc caagtttcta acctgtatca tttttcttat 2160
ctcaataact tcttttaaca tttctcacaa agcagatcta ctggccacag aatgcctcaa 2220
ttttcatttg tctgagaaaa ccttatttct ccttcacttt tgaaagataa ttttgtaggg 2280
tacagaattc taggttgtag gtttttttccc ctcaaagtga aatatttcat tccactcttt 2340
tcttctttgt atggtatctg agaagaagtc agatgtaatt cttatcatta ttacttaaaa 2400
gattgcttct gttcctttct ctcttctcct tcccttcttt ccttctctgt atattacacc 2460
ttttatagtt gccccatatt tcttagatat tatgttttgg ttttcttctg tgtttttttc 2520
tttgattctc agttttagaa gtctctattt atatatctgc aatcgcaggg attctttcct 2580
ctgccatgtc cagtctacta ataagccctt acagacattg ttgacttctg ttccagtgtt 2640
tttgatctct agcatttctc tgattatttc ttggaattgc catctgtcta cttacattac 2700
caacctattc ttgtgtgttg tcttatcata gtaattgcag ttgttttaat ttcataggta 2760
ttgtaatttc aacatctcta ccatatttga cattgattct gatgcttgct ctgtcttatc 2820
aagctatgtt tttgtctttt agtgtgactt ctaatttttt gttgaaagcc aggcatgatg 2880
tactgagtga aagaaactca atacattgta atgtgacgat aagagttcag ggaagtgaa 2940
gcattctata gtcctatagc aggtctcggc cttttagtga gcctgtgcct atgaacggtg 3000
actttcaaca agtgcttttc attccactct tttcctgtcc ttaagtggga caagatcact 3060
gggggggggc tagaattggg tatttccctt ctccaatgta gaagctaaag agagggctgg 3120
agttgggtat ttttcttccc ctgtatggaa agctagaggc agttaaattt ggatattttc 3180
cttcttctaa ttcagttagg ctgcgacaaa aatcccgaca gtttaggctc taatattata 3240
aaataatttc tcttgagtat aggccttatt aagaacacta tactctgatg gagctgaggg 3300
ggagttttct ctgatattca ctgcgagaac ctcgtagagc tccaggaagc aaaactcaca 3360
aaagtgtggg agtcttccag aatttttcct ttgcagactt atctgcactg aacctccaga 3420
aattcatcaa ttacagttca ggttttccta cccaggtact ggttttcatg gaggtttctg 3480
cctgtgcatt tctgctccag taagttgttc ttcttgtatg gtctgtcttt caaatttttt 3540
aagtagggtt atgacctgtc gcctcacttc tctgacagtt ctgagagtgt tgatttttca 3600
gtttgcttag atttttactt gtttttagga tgaagtgaca atttccaagc tcctccctga 3660
catgccagat cagaaactga aagtcctaag cctcatattc tgtgcgtggg tatgttcaca 3720
tcctgcctgc tccagtgccc ccacctcaca ctctctttcc cttccttgtc cccttgtgag 3780
atttctaggt ccaatacaaa gactgtgttc aactcattca actacttggc tcatctgagt 3840
attataatga acaatcacaa aaaaaaatga agtaaaagaa aaatccatca aagaattgag 3900
atatttgaga aaaagaaagg agatcagtgt tttataaaac ttagaaatag atttttaaag 3960
tgtttcttca ttgacttatg tgaaaggact tttcttaatt taacaaatta tgtgctttcg 4020
tttatagcct caaaacttct tgtgtagcta agaatgggta aataatcagg ctttactaaa 4080
ggactaacgt aaagatcttc tgtaagtaac atttctgcta ctcaaggaag agataaactt 4140
catggcataa ccttgccaaa gtatactaag aataaccctg acacaaagct ctttttttcag 4200
ccaacatgcc atgaaagaaa gaagacaagg ggtgatctcc actctctaag tgaaccacta 4260
aacccaccaa agaagaaacg agggaaatag aaagaggacc cttgcctgag ataatggatc 4320
tgtatgtatg agtagtagaa ccctgctcaa agtacaagga agggaaaaaa aagttagttt 4380
atttggaatt ttggacatta agagtcttta ttgttcattt tcttttaact cacatgaatg 4440
gcttatcact tcaattaata aatatttcat ttcttttcaa tctatattca tgaaacaaat 4500
ctgaaatgaa cagtgcaaca tgtgaatgtt tagaacatta taaaattaaa cacaaaatct 4560
gtctggcaat cttcctagca tcttaggaaa aaagttgaca aaatttcaag cagcagaagg 4620
gggcagtaaa actcaacaga aagctctgga agatttttaa gattcttcct tattttcttt 4680
tcatgtagag tatttcccaa caaatttcag acgctaatag aaattttgta caacagatcc 4740
atatatttgc ctaaaataga cacagaaaca ttgatatatg caaacatgag agctataagt 4800
tttacatgat caaaaccttt tttttatggt acacaatagt cacagtactt ttccatataa 4860
aacaggttta gtggtcttaa tttagtttgg cacatttaat acactcccat gaccagcatc 4920
ccaaatgtac ctatccgttt tattttattg tctcagaatt gtcagttatt taataaatta 4980
tgtaactttt ttccttatgc tcagatttgc acttctttct aaaactctgc ccatccttaa 5040
agtcccagat tctccttgaa cttttttttt tgactttcca agtacatgga actcttcact 5100

ctatcctgct atataagtga cagaatttcc actatgggat agatggagtt caattccttt 5160
gagtttaaaa taatctaaat ataattattc cttatgccct gtttttccct cacttttgta 5220
tccaaatctc ttttcagaca acagaacaat taatgtctga taaggaagac aatgatgatg 5280
atcacttcaa aataagcttg aattcaggat tgtaatgtaa aattttagta ctctctcaca 5340
gtatggattc taacatggct tctaacccaa actaacatta gtagctctaa ctataaactt 5400
caaatttcag tagatgcaac ctactccttt aaaatgaaac agaagattga aattattaaa 5460
ttatcaaaaa gaaaatgatc cacgctctta gttgaaattt catgtaagat tccatgcaat 5520
aaataggagt gccataaatg gaatgatgaa atatgactag aggaggagaa aggcttccta 5580
gatgagatgg aattttagtc atccgtgtct catgaagaat cagatgtgta cactaagcaa 5640
aacagttaaa aaaaaaacct ccaagtgagt ctcttattta tttttttctt ataagacttc 5700
tacaaattga ggtacctggt gtagttttat ttcaggtttt atgctgtcat tttcctgtaa 5760
tgctaaggac ttaggacata actgaatttt ctattttcca cttcttttct ggtgtgtgtg 5820
tatatatata tgtatatata cacacacaca tatacatata tatattttta gtatctcacc 5880
ctcacatgct cctccctgag cactacccat gatagatgtt aaacaaaagc aaagatgaaa 5940
ttccaactgt taaaatctcc cttccatcta attaattcct catccaacta tgttccaaaa 6000
cgagaataga aaattagccc caataagccc aggcaactga aaagtaaatg ctatgttgta 6060
ctttgatcca tggtcacaac tcataatctt ggaaaagtgg acagaaaaga caaaagagtg 6120
aactttaaaa ctcgaattta ttttaccagt atctcctatg aagggctagt aaccaaaata 6180
atccacgcat cagggagaga aatgccttaa ggcatacgtt ttggacattt agcgtccctg 6240
caaattctgg ccatcgccgc ttcctttgtc catcagaagg caggaaactt tatattggtg 6300
acccgtggag ctcacattaa ctatttacag ggtaactgct taggaccagt attatgagga 6360
gaatttacct ttcccgcctc tctttccaag aaacaaggag ggggtgaagg tacggagaac 6420
agtatttctt ctgttgaaag caacttagct acaaagataa attacagcta tgtacactga 6480
aggtagctat ttcattccac aaaataagag ttttttaaaa agctatgtat gtatgtcctg 6540
catatagagc agatatacag cctattaagc gtcgtcacta aaacataaaa catgtcagcc 6600
tttcttaacc ttactcgccc cagtctgtcc cgacgtgact tcctcgaccc tctaaagacg 6660
tacagaccag acacggcggc ggcggcggga gaggggattc cctgcgcccc cggacctcag 6720
ggccgctcag attcctggag aggaagccaa gtgtccttct gccctccccc ggtatcccat 6780
ccaaggcgat cagtccagaa ctggctctcg gaagcgctcg ggcaaagact gcgaagaaga 6840
aaagacatct ggcggaaacc tgtgcgcctg gggcggtgga actcggggag gagagggagg 6900
gatcagacag gagagtgggg actaccccct ctgctcccaa attggggcag cttcctgggt 6960
ttccgatttt ctcatttccg tgggtaaaaa accctgcccc caccgggctt acgcaatttt 7020
tttaagggga gaggagggaa aaatttgtgg ggggtacgaa aaggcggaaa gaaacagtca 7080
tttcgtcaca tgggcttggt tttcagtctt ataaaaagga aggttctctc ggttagcgac 7140
caattgtcat acgacttgca gtgagcgtca ggagcacgtc caggaactcc tcagcagcgc 7200
ctccttcagc tccacagcca gacgccctca gacagcaaag cctacccccc gcgccgcgcc 7260
ctgcccgaag ctt 7273

<210> 25
<211> 4465
<212> DNA
<213> Homo sapiens

<400> 25
caattgtcat acgacttgca gtgagcgtca ggagcacgtc caggaactcc tcagcagcgc 60
ctccttcagc tccacagcca gacgccctca gacagcaaag cctacccccg cgccgcgccc 120
tgcccgccgc tcggatgctc gcccgcgccc tgctgctgtg cgcggtcctg gcgctcagcc 180
atacagcaaa tccttgctgt tcccacccat gtcaaaaccg aggtgtatgt atgagtgtgg 240
gatttgacca gtataagtgc gattgtaccc ggacaggatt ctatggagaa aactgctcaa 300
caccggaatt tttgacaaga ataaaattat ttctgaaacc cactccaaac acagtgcact 360
acatacttac ccacttcaag ggattttgga acgttgtgaa taacattccc ttccttcgaa 420
atgcaattat gagttatgtc ttgacatcca gatcacattt gattgacagt ccaccaactt 480
acaatgctga ctatggctac aaaagctggg aagccttctc taacctctcc tattatacta 540
gagcccttcc tcctgtgcct gatgattgcc cgactccctt gggtgtcaaa ggtaaaaagc 600
agcttcctga ttcaaatgag attgtggaaa aattgcttct aagaagaaag ttcatccctg 660
atccccaggg ctcaaacatg atgtttgcat tctttgccca gcacttcacg catcagtttt 720
tcaagacaga tcataagcga gggccagctt tcaccaacgg gctgggccat ggggtggact 780
taaatcatat ttacggtgaa actctggcta gacagcgtaa actgcgcctt ttcaaggatg 840
gaaaaatgaa atatcagata attgatggag agatgtatcc tcccacagtc aaagatactc 900
aggcagagat gatctaccct cctcaagtcc ctgagcatct acggtttgct gtggggcagg 960
aggtctttgg tctggtgcct ggtctgatga tgtatgccac aatctggctg cgggaacaca 1020
acagagtatg cgatgtgctt aaacaggagc atcctgaatg gggtgatgag cagttgttcc 1080
agacaagcag gctaatactg ataggagaga ctattaagat tgtgattgaa gattatgtgc 1140
aacacttgag tggctatcac ttcaaactga aatttgaccc agaactactt ttcaacaaac 1200
aattccagta ccaaaatcgt attgctgctg aatttaacac cctctatcac tggcatcccc 1260
ttctgcctga cacctttcaa attcatgacc agaaatacaa ctatcaacag tttatctaca 1320
acaactctat attgctggaa catggaatta cccagtttgt tgaatcattc accaggcaaa 1380
ttgctggcag ggttgctggt ggtaggaatg ttccacccgc agtacagaaa gtatcacagg 1440
cttccattga ccagagcagg cagatgaaat accagtcttt taatgagtac cgcaaacgct 1500

ttatgctgaa gccctatgaa tcatttgaag aacttacagg agaaaaggaa atgtctgcag 1560
agttggaagc actctatggt gacatcgatg ctgtggagct gtatcctgcc cttctggtag 1620
aaaagcctcg gccagatgcc atctttggtg aaaccatggt agaagttgga gcaccattct 1680
ccttgaaagg acttatgggt aatgttatat gttctcctgc ctactggaag ccaagcactt 1740
ttggtggaga agtgggtttt caaatcatca acactgcctc aattcagtct ctcatctgca 1800
ataacgtgaa gggctgtccc tttacttcat tcagtgttcc agatccagag ctcattaaaa 1860
cagtcaccat caatgcaagt tcttcccgct ccggactaga tgatatcaat cccacagtac 1920
tactaaaaga acgttcgact gaactgtaga agtctaatga tcatatttat ttatttatat 1980
gaaccatgtc tattaattta attatttaat aatatttata ttaaactcct tatgttactt 2040
aacatcttct gtaacagaag tcagtactcc tgttgcggag aaaggagtca tacttgtgaa 2100
gacttttatg tcactactct aaagattttg ctgttgctgt taagtttgga aaacagtttt 2160
tattctgttt tataaaccag agagaaatga gttttgacgt ctttttactt gaatttcaac 2220
ttatattata agaacgaaag taaagatgtt tgaatactta aacactatca caagatggca 2280
aaatgctgaa agtttttaca ctgtcgatgt ttccaatgca tcttccatga tgcattagaa 2340
gtaactaatg tttgaaattt taaagtactt ttggttattt ttctgtcatc aaacaaaaac 2400
aggtatcagt gcattattaa atgaatattt aaattagaca ttaccagtaa tttcatgtct 2460
actttttaaa atcagcaatg aaacaataat ttgaaatttc taaattcata gggtagaatc 2520
acctgtaaaa gcttgtttga tttcttaaag ttattaaact tgtacatata ccaaaaagaa 2580
gctgtcttgg atttaaatct gtaaaatcag atgaaatttt actacaattg cttgttaaaa 2640
tattttataa gtgatgttcc tttttcacca agagtataaa cctttttagt gtgactgtta 2700
aaacttcctt ttaaatcaaa atgccaaatt tattaaggtg gtggagccac tgcagtgtta 2760
tctcaaaata agaatatttt gttgagatat tccagaattt gtttatatgg ctggtaacat 2820
gtaaaatcta tatcagcaaa agggtctacc tttaaaataa gcaataacaa agaagaaaac 2880
caaattattg ttcaaattta ggtttaaact tttgaagcaa actttttttt atccttgtgc 2940
actgcaggcc tggtactcag attttgctat gaggttaatg aagtaccaag ctgtgcttga 3000
ataacgatat gttttctcag attttctgtt gtacagttta atttagcagt ccatatcaca 3060
ttgcaaaagt agcaatgacc tcataaaata cctcttcaaa atgcttaaat tcatttcaca 3120
cattaatttt atctcagtct tgaagccaat tcagtaggtg cattggaatc aagcctggct 3180
acctgcatgc tgttcctttt cttttcttct tttagccatt ttgctaagag acacagtctt 3240
ctcatcactt cgtttctcct attttgtttt actagtttta agatcagagt tcactttctt 3300
tggactctgc ctatattttc ttacctgaac ttttgcaagt tttcaggtaa acctcagctc 3360
aggactgcta tttagctcct cttaagaaga ttaaaagaga aaaaaaaagg cccttttaaa 3420
aatagtatac acttatttta agtgaaaagc agagaatttt atttatagct aattttagct 3480
atctgtaacc aagatggatg caaagaggct agtgcctcag agagaactgt acggggtttg 3540
tgactggaaa aagttacgtt cccattctaa ttaatgccct ttcttattta aaaacaaaac 3600
caaatgatat ctaagtagtt ctcagcaata ataataatga cgataatact tcttttccac 3660
atctcattgt cactgacatt taatggtact gtatattact taatttattg aagattatta 3720
tttatgtctt attaggacac tatggttata aactgtgttt aagcctacaa tcattgattt 3780
ttttttgtta tgtcacaatc agtatatttt ctttggggtt acctctctga atattatgta 3840
aacaatccaa agaaatgatt gtattaagat ttgtgaataa atttttagaa atctgattgg 3900
catattgaga tatttaaggt tgaatgtttg tccttaggat aggcctatgt gctagcccac 3960
aaagaatatt gtctcattag cctgaatgtg ccataagact gaccttttaa aatgttttga 4020
gggatctgtg gatgcttcgt taatttgttc agccacaatt tattgagaaa atattctgtg 4080
tcaagcactg tgggttttaa tatttttaaa tcaaacgctg attacagata atagtattta 4140
tataaataat tgaaaaaaat tttcttttgg gaagagggag aaaatgaaat aaatatcatt 4200
aaagataact caggagaatc ttctttacaa ttttacgttt agaatgttta aggttaagaa 4260
agaaatagtc aatatgcttg tataaaacac tgttcactgt tttttttaaa aaaaaaactt 4320
gatttgttat taacattgat ctgctgacaa aacctgggaa tttgggttgt gtatgcgaat 4380
gtttcagtgc ctcagacaaa tgtgtattta acttatgtaa aagataagtc tggaaataaa 4440
tgtctgttta tttttgtact attta 4465

## Description

### BACKGROUND OF THE INVENTION

This invention relates to the interrogation of methylated genes in concert with other diagnostic methods and kits for use with these methods.

In higher order eukaryotes DNA is methylated only at cytosines located 5' to guanosine in the CpG dinucleotide. This modification has important regulatory effects on gene expression, especially when it involves CpG rich areas (CpG islands) located in gene promoter regions. Aberrant methylation of normally unmethylated CpG islands is a frequent event in immortalized and transformed cells and has been associated with transcriptional inactivation of certain tumor suppressor genes or genes otherwise associated with the amelioration of certain human cancers.

A number of potential methylation markers have recently been disclosed. Glutathione S-transferases (GSTs) are exemplary proteins in which the methylation status of the genes that express them can have important prognostic and diagnostic value for prostate cancer. The proteins catalyze intracellular detoxification reactions, including the inactivation of electrophilic carcinogens, by conjugating chemically-reactive electrophiles to glutathione (C. B. Pickett, et al., Annu. Rev. Blocbern., 58:743, 1989; B. Coles, et al., CRC Crit. Rev. Biochem. Mol. Biol., 25:47, 1990; T. H. Rushmore, et al., J. Biol. Chem. 268:11475, 1993). Human GSTs, encoded by several different genes at different loci, have been classified into four families referred to as alpha, mu, pi, and theta (B. Mannervik, et al., Biochem. J., 282:305, 1992). Decreased GSTP1 expression resulting from epigenetic changes is often related to prostate and hepatic cancers.

The S100 proteins are calcium-binding proteins that are implicated in, among other things, tumerigenesis. The family includes S100A2, S100A4, S100A5, S100A6, S100A8, S100A9, and S100A11, which have all been shown to bear some relationship to tumor development though precisely what that role is has not been clear. S 100A6 (calcylin) expression appears to fall off in prostate cancer development. S100A2 has been shown to exhibit lessened expression in breast, lung, and prostate cancer as well. This is believed to be due to hypermethylation of the gene promoter but the picture is not clear since hypermethylation is also seen in non-malignant prostate epithelium and BPH.

Sampling and sample preparation are important factors in epigenetic testing. Every sample source has its issues. Even biopsy samples taken directly from the affected tissue are known to present the possibility of false negative results due to uneven distribution of affected cells. Urine is a desirable sample because it can be obtained less invasively than many other potential samples. The number and concentration of prostate cancer cells shed into urine can be extremely variable depending on a host of factors such as when the urine is collected, whether it is collected pursuant to prostate massage, and the presence and effect of nucleases and reagents and methods for minimizing their effect.

While some have proposed prostate cancer testing on urine samples, actually producing such a test has proven difficult. First, it is presumed that the basis for such a test is the shedding of cancer cells from the tumor or lesion into the urinary system. Little is actually known about this process. It also seems likely that analyte concentrations could vary much more dramatically than in other samples such as tissue biopsy and even serum samples depending on a wide range of physiological and environmental factors such as the degree to which the patient is hydrated. The stability of the analyte in the matrix is also not well understood in light of the presence of nucleases and a wide variety of other substances that can affect nucleic acids. Sample preparation for a number of other urine assays use spun down samples referred to as sediments. Whether this makes sense for methylation markers cannot be supposed a priori.

Preparation of the patient and pretreatment options are also not well understood. Digital rectal examinations (DRE) are standard diagnostic procedures for determining prostate health in which the physician notes anatomical abnormalities. In the past the outcome of the DRE would be used to determine whether a biopsy or other diagnostic or therapeutic procedure would be necessary. Whether and to what extent procedures such as the DRE or related prostate massage causes cells to slough so that they would then be detected in the subsequent diagnostic procedure was unclear. Procedurally, DRE and digital rectal massage and the time in which they are performed can differ greatly further adding to the list of unknowns in this area.

### SUMMARY OF THE INVENTION

In one aspect of the invention, a method for characterizing prostate cancer in a patient comprises assaying GSTP1 methylation and one or more control genes in urine within three days of its collection. The assay is considered positive for prostate cancer if the degree of methylation of the GSTP1 exceeds a pre-determined value and is considered negative for prostate cancer if the pre-determined value is not exceeded.

In another aspect of the invention, a method for characterizing prostate cancer in a patient comprises assaying GSTP1 methylation, one or more control genes, and the S100 gene in urine within three days of its collection. A normalized value of GSTP1 is determined by comparison of the GSTP1 methylation assay value to that of the S100 methylation assay value. The assay is considered positive for prostate cancer if the normalized methylation assay value exceeds a pre-determined value and is considered negative for prostate cancer if the pre-determined value is not exceeded.

In yet another aspect of the invention, a method for characterizing prostate cancer in a patient comprises assaying GSTP1 methylation and one or more control genes conducted as a nested PCR reaction. The assay is considered positive for prostate cancer if the degree of methylation of the GSTP1 exceeds a pre-determined value and is considered negative for prostate cancer if the pre-determined value is not exceeded.

In yet another aspect of the invention, methylation of the following panels of genes is detected:
a. GSTP1, APC.
b. GSTP1, APC, S100A2.
c. GSTP1, RARβ2.
d. GSTP1, RAR β2, S100A2.

The panels may also include control genes.

In yet another aspect of the invention, methylation status is determined via quantitative real time PCR.

In yet another aspect, the invention is a kit useful for the detection of a methylated nucleic acid. The kit includes one or more containers; a first container containing a reagent that modifies unmethylated cytosine and a second container containing a reagent that primes amplification of CpG-containing nucleic acid, wherein the reagent distinguishes between modified methylated and nonmethylated nucleic acid. The kit contains instructions to conduct the assay on patients suspected of having prostate cancer.

In yet another aspect of the invention, the kit includes a reaction vessel having separate components into which primers are initially stored and wherein during use of the kit, the primers are exuded into a reaction chamber according to a set sequence such that methylation status can be properly assessed. The primers can be for conducting nested amplification reactions.

### DETAILED DESCRIPTION OF THE INVENTION

The urine-based assay of this invention is preferably conducted on patients who have had a PSA assay with ambiguous or difficult to determine results (most preferably 2.5-4.0 ng/ml). A negative result using the assay of this invention (in the absence of other clinical indicia) can spare the patient of more invasive testing such as with a biopsy procedure. Thus, viewed most inclusively, one method according to the invention involves first conducting a PSA test in a patient and then conducting the assay described more fully below on those patients having a PSA level assayed at 2.5-4.0 ng/ml.

The assays of the invention detect hypermethylation of nucleic acids that correspond to particular genes whose methylation status correlates with prostate cancer. A nucleic acid corresponds to a gene whose methylation status correlates with prostate cancer when methylation status of such a gene provides information about prostate cancer and the sequence is a coding portion of the gene or its complement, a representative portion of the gene or its complement, a promoter or regulatory sequence for the gene or its complement, a sequence that indicates the presence of the gene or its complement, or the full length sequence of the gene or its complement. Such nucleic acids are referred to as Markers in this specification. Markers correspond to the following genes only: GSTP1 (Seq. ID. No. 17), APC (Promoter= Seq. ID. No. 18, Gene= Seq. ID. No. 19), RARβ2 (Seq. ID No. 20), S100A2 (Seq. ID. No. 21). Other sequences of interest include constitutive genes useful as assay controls such as beta-Actin (Seq. ID. No.22 and 23) and PTGS2 (Promoter= Seq. ID. No.24, Gene= Seq. ID. No. 25).

Assays for detecting hypermethylation include such techniques as MSP and restriction endonuclease analysis. The promoter region is a particularly noteworthy target for detecting such hypermethylation analysis. Sequence analysis of the promoter region of GSTP1 shows that nearly 72% of the nucleotides are CG and about 10% are CpG dinucleotides.

The invention includes determining the methylation status of certain regions of the Markers in urine or urethral washes and in which the DNA associated with prostate cancer is amplified and detected. Since a decreased level of the protein encoded by the Marker (i.e., less transcription) is often the result of hypermethylation of a particular region such as the promoter, it is desirable to determine whether such regions are hypermethylated. This is seen most demonstrably in the case of the GSTP1 gene and in the panels indicated in the Summary of the Invention. A nucleic acid probe or reporter specific for certain Marker regions is used to detect the presence of methylated regions of the Marker gene. Hypermethylated regions are those that are methylated to a statistically significant greater degree in samples from diseased tissue as compared to normal tissue.

As noted above, urine is the matrix in which the assays of this invention are conducted. Most preferably, it is collected after prostate massage and stored at 4C until it can be sedimented. It is most preferably spun down within 4 hours.

Prostatic massage, when conducted in conjunction with the methylation analyses of the invention, is best conducted as follows: the gland is pressed firmly enough to depress the surface from the base to the apex and from the lateral to the median line for each lobe to ensure the release of sufficient number of prostate cells. It is most preferred that this massage procedure is conducted for 20 seconds or less.

Some of the primers/probes or reporter reagents of the invention are used to detect methylation of expression control sequences of the Marker genes. These are nucleic acid sequences that regulate the transcription and, in some cases, translation of the nucleic acid sequence. Thus, expression control sequences can include sequences involved with promoters, enhancers, transcription terminators, start codons (i.e., ATG), splicing signals for introns, maintenance of the correct reading frame of that gene to permit proper translation of the mRNA, and stop codons.

The GSTP1 promoter is the most preferred Marker. It is a polynucleotide sequence that can direct transcription of the gene to produce a glutathione-s-transferase protein. The promoter region is located upstream, or 5' to the structural gene. It may include elements which are sufficient to render promoter-dependent gene expression controllable for cell-type specific, tissue-specific, or inducible by external signals or agents; such elements may be located in the 5' or 3' regions of the of the polynucleotide sequence.

One method of the invention includes contacting a target cell containing a Marker with a reagent that binds to the nucleic acid. The target cell component is a nucleic acid such as DNA extracted from urine by cell lysis and purification (column or solution based) yielding pure DNA that is devoid of proteins. The reagents include components that prime and probe PCR or MSP reactions and detect the target sequence. These reagents can include priming sequences combined with or bonded to their own reporter segments such as those referred to as Scorpion reagents or Scorpion reporters and described in US Patents 6,326,145 and 6,270,967 to Whitcombe et. al*.* (incorporated herein by reference in their entirety). Though they are not the same, the terms "primers" and "priming sequences" may be used in this specification to refer to molecules or portions of molecules that prime the amplification of nucleic acid sequences.

One sensitive method of detecting methylation patterns involves combining the use of methylation-sensitive enzymes and the polymerase chain reaction (PCR). After digestion of DNA with the enzyme, PCR will amplify from primers flanking the restriction site only if DNA cleavage was prevented by methylation. The PCR primers of the invention are designed to target the promoter and transcription region that lies approximately between -71 and+59 bp according to genomic positioning number of M24485 (Genbank) from the transcription start site of GSTP1.

The method of the invention can also include contacting a nucleic acid-containing specimen with an agent that modifies unmethylated cytosine; amplifying the CpG-containing nucleic acid in the specimen by means of CpG-specific oligonucleotide primers; and detecting the methylated nucleic acid. The preferred modification is the conversion of unmethylated cytosines to another nucleotide that will distinguish the unmethylated from the methylated cytosine. Preferably, the agent modifies unmethylated cytosine to uracil and is sodium bisulfite, however, other agents that modify unmethylated cytosine, but not methylated cytosine can also be used. Sodium bisulfite (NaHSO₃) modification is most preferred and reacts readily with the 5,6-double bond of cytosine, but poorly with methylated cytosine. Cytosine reacts with the bisulfite ion to form a sulfonated cytosine reaction intermediate susceptible to deamination, giving rise to a sulfonated uracil. The sulfonate group can be removed under alkaline conditions, resulting in the formation of uracil. Uracil is recognized as a thymine by Taq polymerase and therefore upon PCR, the resultant product contains cytosine only at the position where 5-methylcytosine occurs in the starting template. Scorpion reporters and reagents and other detection systems similarly distinguish modified from unmodified species treated in this manner.

The primers used in the invention for amplification of a CpG-containing nucleic acid in the specimen, after modification (e.g., with bisulfite), specifically distinguish between untreated DNA, methylated, and non-methylated DNA. In methylation specific PCR (MSPCR), primers or priming sequences for the non-methylated DNA preferably have a T in the 3' CG pair to distinguish it from the C retained in methylated DNA, and the complement is designed for the antisense primer. MSP primers or priming sequences for non-methylated DNA usually contain relatively few Cs or Gs in the sequence since the Cs will be absent in the sense primer and the Gs absent in the antisense primer (C becomes modified to U (uracil) which is amplified as T (thymidine) in the amplification product).

The primers of the invention are oligonucleotides of sufficient length and appropriate sequence so as to provide specific initiation of polymerization on a significant number of nucleic acids in the polymorphic locus. When exposed to appropriate probes or reporters, the sequences that are amplified reveal methylation status and thus diagnostic information.

Preferred primers are most preferably eight or more deoxyribonucleotides or ribonucleotides capable of initiating synthesis of a primer extension product, which is substantially complementary to a polymorphic locus strand. Environmental conditions conducive to synthesis include the presence of nucleoside triphosphates and an agent for polymerization, such as DNA polymerase, and a suitable temperature and pH. The priming segment of the primer or priming sequence is preferably single stranded for maximum efficiency in amplification, but may be double stranded. If double stranded, the primer is first treated to separate its strands before being used to prepare extension products. The primer must be sufficiently long to prime the synthesis of extension products in the presence of the inducing agent for polymerization. The exact length of primer will depend on factors such as temperature, buffer, cations, and nucleotide composition. The oligonucleotide primers most preferably contain about 12-20 nucleotides although they may contain more or fewer nucleotides, preferably according to well known design guidelines or rules.

Primers are designed to be substantially complementary to each strand of the genomic locus to be amplified and include the appropriate G or C nucleotides as discussed above.

This means that the primers must be sufficiently complementary to hybridize with their respective strands under conditions that allow the agent for polymerization to perform. In other words, the primers should have sufficient complementarity with the 5' and 3' flanking sequence(s) to hybridize and permit amplification of the genomic locus.

The primers are employed in the amplification process. That is, reactions (preferably, an enzymatic chain reaction) that produce greater quantities of target locus relative to the number of reaction steps involved. In a most preferred embodiment, the reaction produces exponentially greater quantities of the target locus. Reactions such as these include the PCR reaction. Typically, one primer is complementary to the negative (-) strand of the locus and the other is complementary to the positive (+) strand. Annealing the primers to denatured nucleic acid followed by extension with an enzyme, such as the large fragment of DNA Polymerase I (Klenow) and nucleotides, results in newly synthesized + and - strands containing the target locus sequence. The product of the chain reaction is a discrete nucleic acid duplex with termini corresponding to the ends of the specific primers employed.

The primers may be prepared using any suitable method, such as conventional phosphotriester and phosphodiester methods including automated methods. In one such automated embodiment, diethylphosphoramidites are used as starting materials and may be synthesized as described by Beaucage, et at. (Tetrahedron Letters, 22:1859-1862, 1981). A method for synthesizing oligonucleotides on a modified solid support is described in U.S. Pat. No. 4,458,066.
Any nucleic acid specimen taken from urine or urethral wash, in purified or non-purified form, can be utilized as the starting nucleic acid or acids, provided it contains, or is suspected of containing, the specific nucleic acid sequence containing the target locus (e.g., CpG). Thus, the process may employ, for example, DNA or RNA, including messenger RNA. The DNA or RNA may be single stranded or double stranded. In the event that RNA is to be used as a template, enzymes, and/or conditions optimal for reverse transcribing the template to DNA would be utilized. In addition, a DNA-RNA hybrid containing one strand of each may be utilized. A mixture of nucleic acids may also be employed, or the nucleic acids produced in a previous amplification reaction herein, using the same or different primers may be so utilized. The specific nucleic acid sequence to be amplified, i.e., the target locus, may be a fraction of a larger molecule or can be present initially as a discrete molecule so that the specific sequence constitutes the entire nucleic acid.

If the extracted sample is impure, it may be treated before amplification with an amount of a reagent effective to open the cells, fluids, tissues, or animal cell membranes of the sample, and to expose and/or separate the strand(s) of the nucleic acid(s). This lysing and nucleic acid denaturing step to expose and separate the strands will allow amplification to occur much more readily.

Where the target nucleic acid sequence of the sample contains two strands, it is necessary to separate the strands of the nucleic acid before it can be used as the template. Strand separation can be effected either as a separate step or simultaneously with the synthesis of the primer extension products. This strand separation can be accomplished using various suitable denaturing conditions, including physical, chemical or enzymatic means. One physical method of separating nucleic acid strands involves heating the nucleic acid until it is denatured. Typical heat denaturation may involve temperatures ranging from about 80 to 105°C for up to 10 minutes. Strand separation may also be induced by an enzyme from the class of enzymes known as helicases or by the enzyme RecA, which has helicase activity, and in the presence of riboATP, is known to denature DNA. Reaction conditions that are suitable for strand separation of nucleic acids using helicases are described by Kuhn Hoffmann-Berling (CSH-Quantitative Biology, 43:63, 1978). Techniques for using RecA are reviewed in C. Radding (Ann. Rev. Genetics, 16:405-437, 1982). Refinements of these techniques are now also well known.

When complementary strands of nucleic acid or acids are separated, regardless of whether the nucleic acid was originally double or single stranded, the separated strands are ready to be used as a template for the synthesis of additional nucleic acid strands. This synthesis is performed under conditions allowing hybridization of primers to templates to occur. Generally synthesis occurs in a buffered aqueous solution, preferably at a pH of 7-9, most preferably about 8. A molar excess (for genomic nucleic acid, usually about 10⁸:1, primer:template) of the two oligonucleotide primers is preferably added to the buffer containing the separated template strands. The amount of complementary strand may not be known if the process of the invention is used for diagnostic applications, so the amount of primer relative to the amount of complementary strand cannot always be determined with certainty. As a practical matter, however, the amount of primer added will generally be in molar excess over the amount of complementary strand (template) when the sequence to be amplified is contained in a mixture of complicated long-chain nucleic acid strands. A large molar excess is preferred to improve the efficiency of the process.

The deoxyribonucleoside triphosphates dATP, dCTP, dGTP, and dTTP are added to the synthesis mixture, either separately or together with the primers, in adequate amounts and the resulting solution is heated to about 90-100°C for up to 10 minutes, preferably from 1 to 4 minutes. After this heating period, the solution is allowed to cool to room temperature, which is preferable for the primer hybridization. To the cooled mixture is added an appropriate agent for effecting the primer extension reaction (the "agent for polymerization"), and the reaction is allowed to occur under conditions known in the art. The agent for polymerization may also be added together with the other reagents if it is heat stable. This synthesis (or amplification) reaction may occur at room temperature up to a temperature at which the agent for polymerization no longer functions.

The agent for polymerization may be any compound or system that will function to accomplish the synthesis of primer extension products, preferably enzymes. Suitable enzymes for this purpose include, for example, E. coli DNA polymerase 1, Klenow fragment of E. coli DNA polymerase I, T4 DNA polymerase, other available DNA polymerases, polymerase mutants, reverse transcriptase, and other enzymes, including heat-stable enzymes (e.g., those enzymes which perform primer extension after being subjected to temperatures sufficiently elevated to cause denaturating). A preferred agent is Taq polymerase. Suitable enzymes will facilitate combination of the nucleotides in the proper manner to form the primer extension products complementary to each locus nucleic acid strand. Generally, the synthesis will be initiated at the 3' end of each primer and proceed in the 5' direction along the template strand, until synthesis terminates, producing molecules of different lengths. There may be agents for polymerization, however, which initiate synthesis at the 5' end and proceed in the other direction, using the same process as described above.

Most preferably, the method of amplifying is by PCR. Alternative methods of amplification can also be employed as long as the methylated and non-methylated loci amplified by PCR using the primers of the invention is similarly amplified by the alternative means. In one such most preferred embodiment, the assay is conducted as a nested PCR. In nested PCR methods, two or more staged polymerase chain reactions are undertaken. In a first-stage polymerase chain reaction, a pair of outer oligonucleotide primers, consisting of an upper and a lower primer that flank a particular first target nucleotide sequence in the 5' and 3' position, respectively, are used to amplify that first sequence. In subsequent stages, a second set of inner or nested oligonucleotide primers, also consisting of an upper and a lower primer, are used to amplify a smaller second target nucleotide sequence that is contained within the first target nucleotide sequence. The upper and lower inner primers flank the second target nucleotide sequence in the 5' and 3' positions, respectively. Flanking primers are complementary to segments on the 3'-end portions of the double-stranded target nucleotide sequence that is amplified during the PCR process.

The first nucleotide sequence within the region of the gene targeted for amplification in the first-stage polymerase chain reaction is flanked by an upper primer in the 5' upstream position and a lower primer in the 3' downstream position. The first targeted nucleotide sequence, and hence the amplification product of the first-stage polymerase chain reaction, has a predicted base-pair length, which is determined by the base-pair distance between the 5' upstream and 3' downstream hybridization positions of the upper and lower primers, respectively, of the outer primer pair.

At the end of the first-stage polymerase chain reaction, an aliquot of the resulting mixture is carried over into a second-stage polymerase chain reaction. This is preferably conducted within a sealed or closed vessel automatically such as with the "SMART CAP" device from Cepheid. In this second-stage reaction, the products of the first-stage reaction are combined with specific inner or nested primers. These inner primers are derived from nucleotide sequences within the first targeted nucleotide sequence and flank a second, smaller targeted nucleotide sequence contained within the first targeted nucleotide sequence. This mixture is subjected to initial denaturation, annealing, and extension steps, followed by thermocycling as before to allow for repeated denaturation, annealing, and extension or replication of the second targeted nucleotide sequence. This second targeted nucleotide sequence is flanked by an upper primer in the 5' upstream position and a lower primer in the 3' downstream position. The second targeted nucleotide sequence, and hence the amplification product of the second-stage PCR, also has a predicted base-pair length, which is determined by the base-pair distance between the 5' upstream and 3' downstream hybridization positions of the upper and lower primers, respectively, of the inner primer pair.

The amplified products are preferably identified as methylated or non-methylated with a probe or reporter specific to the product as described in US Patent 4,683,195 to Mullis et. al*.,* incorporated herein by reference in its entirety. Advances in the field of probes and reporters for detecting polynucleotides are well known to those skilled in the art. Optionally, the methylation pattern of the nucleic acid can be confirmed by other techniques such as restriction enzyme digestion and Southern blot analysis. Examples of methylation sensitive restriction endonucleases which can be used to detect 5'CpG methylation include SmaI, SacII, EagI, MspI, HpaII, BstUI and BssHII.

In another aspect of the invention a methylation ratio is used. This can be done by establishing a ratio between the amount of amplified methylated species of Marker attained and the amount of amplified reference Marker or non-methylated Marker region amplified. This is best done using quantitative real-time PCR. Ratios above an established or predetermined cutoff or threshold are considered hypermethylated and indicative of having a proliferative disorder such as cancer (prostate cancer in the case of GSTP1). Cutoffs are established according to known methods in which such methods are used for at least two sets of samples: those with known diseased conditions and those with known normal conditions. The reference Markers of the invention can also be used as internal controls. The reference Marker is preferably a gene that is constitutively expressed in the cells of the samples such as Beta Actin.

Established or predetermined values (cutoff or threshold values) are also established and used in methods according to the invention in which a ratio is not used. In this case, the cutoff value is established with respect to the amount or degree of methylation relative to some baseline value such as the amount or degree of methylation in normal samples or in samples in which the cancer is clinically insignificant (is known not to progress to clinically relevant states or is not aggressive). These cutoffs are established according to well-known methods as in the case of their use in methods based on a methylation ratio.

In the most preferred embodiment of the invention, GSTP1 methylation values obtained by MSP or other suitable methods are normalized with S100A2 methylation values determined using the same method. The normalized value is obtained by subtracting the S100A2 assay value from that of the GSTP1 value as shown in Example 7. Other normalization methods can also be used such as generation of a methylation ratio (obtained by converting the Ct value to a copy number for the gene of interest and dividing that copy number by the copy number for beta-actin, obtained in the same manner). When using a normalized value, the cutoff value is determined by first generating a training set in which the cutoff generates optimal sensitivity and specificity and then validating the cutoff in an independent validation set.

The inventive methods and kits can include steps and reagents for multiplexing. That is, more than one Marker can be assayed at a time. But only the following Markers are assayed as part of this invention GSTP1, RAR- β2, APC, and S100A2 along with internal controls such as β-Actin.

Since a decreased level of transcription of the gene associated with the Marker is often the result of hypermethylation of the polynucleotide sequence and/or particular elements of the expression control sequences (e.g., the promoter sequence), primers prepared to match those sequences were prepared. Accordingly, the invention provides methods of detecting or diagnosing a cell proliferative disorder by detecting methylation of particular areas, preferably, within the expression control or promoter region of the Markers. Probes useful for detecting methylation of these areas are useful in such diagnostic or prognostic methods.

The kits of the invention can be configured with a variety of components provided that they all contain at least one primer or probe or a detection molecule (e.g., Scorpion reporter). In one embodiment, the kit includes reagents for amplifying and detecting hypermethylated Marker segments. Optionally, the kit includes sample preparation reagents and /or articles (e.g., tubes) to extract nucleic acids from samples.

In a preferred kit, reagents necessary for one-tube MSP are included such as, a corresponding PCR primer set, a thermostable DNA polymerase, such as Taq polymerase, and a suitable detection reagent(s) such as hydrolysis probe or molecular beacon. In optionally preferred kits, detection reagents are Scorpion reporters or reagents. A single dye primer or a fluorescent dye specific to double-stranded DNA such as ethidium bromide can also be used. The primers are preferably in quantities that yield high concentrations. Additional materials in the kit may include: suitable reaction tubes or vials, a barrier composition, typically a wax bead, optionally including magnesium; necessary buffers and reagents such as dNTPs; control nucleic acid (s) and/or any additional buffers, compounds, co-factors, ionic constituents, proteins and enzymes, polymers, and the like that may be used in MSP reactions. Optionally, the kits include nucleic acid extraction reagents and materials.

### EXAMPLES

### Example 1: Sample Preparation and MSPCR

Prostate samples were obtained from patients with known clinical outcomes.

The methylation assays were conducted as follows. Genomic DNA was modified using a commercially available sodium bisulfite conversion reagent kit (Zymo Research, Orange, CA, USA). This treatment converted all Cytosines in unmethylated DNA into Uracil, whereas in methylated DNA only cytosines not preceding guanine were converted into Uracil. All cytosines preceeding guanine (in a CpG dinucletide) remained as cytosine. The assays are described more fully below.

### a. Sedimentation: Sedimented urine samples were obtained as follows.

50-ml Falcon tubes containing the urine were centrifuged at 3000g on VRX Sorvall centrifuge for 10 minutes at +4 degrees C. Supernatant was removed leaving ~5ml on top of the pellet. The tubes were then spun down again (3000g for 5 minutes) in order to discard the remaining supernatant (using 1 ml tips). Urine sediment was then rinsed with 20 mL cold (4C) PBS, spun down again (3000g for 5 minutes), and the residual supernatant was aspirated. Samples were then stored at -20C.

### b. Cell Lysis and DNA extraction:

The cells in the sediment were then lysed as follows. 700 µl Cell Lysis Solution was added to each sample containing a urine cell pellet. The lysate was then transferred in a 2.0 ml microfuge tube and 3 µl Proteinase K Solution (20 mg/ml) was added to the lysate, mixed by inverting 25 times, and incubated for one hour to overnight at 55°C.

Samples were cooled to room temperature by placing at 20°C (heat block) for 10 minutes. 300 µl Protein Precipitation Solution was then added to the lysate which was then vortexed vigorously at high speed for 20 seconds The samples were placed into an ice bath for 5 minutes and centrifuged at (16000 RPM) for 5 minutes. The precipitated proteins formed a tight pellet. The supernatant was then transferred to a new 2.0 ml tube with the precipitation steps repeated.

The supernatant containing the DNA was then transferred into a clean 2.0 ml microfuge tube and centrifugation was repeated (16000 RPM for 3 minutes) with the supernatant again transferred into a clean 2.0 ml microfuge tube containing 900 µl 100% isopropanol and 2 µl Glycogen 20 mg/ml. The sample was mixed by inverting gently 50 times and kept at room temperature for at least 10-15 minutes on the rocker and then cooled to - 20C. The sample was then centrifuge at 16000 RPM for 5 minutes. The DNA was then visible as a small white pellet. Supernatant was removed with the lml-pipet and the sample was ccentrifuge at (16000 RPM) for 60 seconds. Remaining supernatant was removed with a 100µl-pipet. 900 µl 70% ethanol was added and the tube was inverted 10 times to wash the DNA pellet followed by another centrifugation at 16000 RPM for 1 minute. Ethanol was discarded with the 1ml-pipet followed by another centrifugation at (16000 RPM) for 60 seconds.The remaining supernatant was discarded with the 100µl-pipet and the sample was allowed to air dry 10-15 minutes.

45 µl LoTE buffer was added to the dried samples and the DNA was rehydrated by incubating at 65°C for 1 hour shaking at 1100 rpm and overnight at 20°C shaking at 1100 rpm. The DNA was stored in a clearly labelled tube at -80°C.

### c. Bisulfite modification:

DNA Samples were then modified using EZ-DNA methylation kit from ZymoResearch (Cat. No D5001) as follows.

24 ml absolute Ethanol was added to the M-Wash buffer Concentrate to make the final M-Wash buffer. 5 ul of M-Dilution Buffer directly to 45 µl of the DNA sample. This mixture was mixed by pipetting up and down and then spun briefly followed by incubation at 37°C for 15 minutes in a heat block with shaking at 1100 rpm. During the incubation, CT Conversion Reagent was prepared by adding 750 µl Baker Water and 210 µl of M-Dilution Buffer. It was then mixed by vortexing for 1 minute every 2 minutes for a total of 10 minutes. After the above incubation, 100 µl of the prepared CT Conversion Reagent (after briefly spinning) was added to each sample which was then vortexed lightly and spun briefly. The sample was then incubated at 70°C for 3 hour with the heating block (shaking at 1100 rpm) covered with aluminum foil.

The sample was then spun down briefly and set on ice for 10 minutes. 400 µl of M-Binding buffer was added to the sample which was mixed by pipetting up and down. All the supernatant was loaded into a Zymo-Spin Column which was placed into a 2 ml collection tube. The tube was centrifuged at maximum speed for 15 - 30 seconds the flow-through discarded. 200 µl of M-Wash Buffer was added to the column which was centrifuge at maximum speed for 15 - 30 seconds again with the flow-through again discarded.

200 µl of M-Desulphonation Buffer was added to the column and let to stand at room temperature for 15 minutes followed by centrifugation at maximum speed for 15 -30 seconds with the flow-through discarded. This procedure was followed three times with the last centrifugation step lasting 30 seconds. The column was then placed onto a clean 1.5 ml tube to which 50 ul of M-elution buffer was added. The columns were then let to stand for 1 min at RT followed by centrifugation at maximum speed for 1 minute to elute the DNA. The eluted DNA was labeled and stored as 'BT modified' at -80°C.

MSPCR assays were then set up with the following primers and probes:

**Outer PCR primers**

| | | |
|---|---|---|
| GSTP1_332_U18 | Seq ID No. 1 | TCGGGGATTTTAGGGCGT |
| GSTP1_513_L21 | Seq ID No. 2 | ACGAAAACTACGACGACGAAA |
| Actin_309_U24 | Seq ID No. 5 | GATATAAGGTTAGGGATAGGATAG |
| Actin_501_L22 | Seq ID No. 6 | AACCAATAAAACCTACTCCTCC |
| APC_Outer_692_U19 | Seq ID No. 9 | CCCTATACCCCACTACGAA |
| APC_Outer_830_L25 | Seq ID No. 10 | GGCGGGTTGTATTAATATAGTTATA |
| RARB2_Outer_16_U25 | Seq ID No. 13 | GGAAGTGAGTTGTTTAGAGGTAGGA |
| RARB2_Outer_239_L25 | Seq ID No. 14 | TCCAAACTTACTCGACCAATCCAAC |

**Inner PCR Scorpion probe/primer sets**

| **Description** | **Sequence** | **Seq ID No** |
|---|---|---|
| GSTP1 Scorpion | FAM-CGCACGGCGAACTCCCGCCGACGTGCG BHQ-HEG-TGTAGCGGTCGTCGGGGTTG | 3 |
| GSTPi Reverse Primer | 5' GCCCCAATACTAAATCACGACG 3' | 4 |
| Actin Scorpion | Q670-CCGCGCATCACCACCCCACACGCGCGG- BHQ2-HEG-GGAGTATATAGGTTGGGGAAGTTTG | 7 |
| Actin Reverse Primer | 5' AACACACAATAACAAACACAAATTCAC 3' | 8 |
| APC Scorpion | Texas Red - GCCGGCGGGTTTTCGACGGGCCGGC- BHQ2-HEG-CGAACCAAAACGCTCCCCA | 11 |
| APC Lower Primer | GTCGGTTACGTGCGTTTATATTTAG | 12 |
| RARB2 Scorpion | Q570 - CGGCGCCCGACGATACCCAAAGCGCCG- BHQ2-HEG- AACGCGAGCGATTCGAGTAG | 15 |
| RARB2 Lower Primer | CTTACAAAAAAACCTTCCGAATACG | 16 |

| | | |
|---|---|---|
| BHQ= Black Hole Quencher reporter molecule. HEG= hexaethylene glycol | | |

Nested PCR reactions were conducted using "SMARTCAP" tubes (Cepheid) and the "SMARTCYCLER" (Cepheid) PCR analyzer as follows.

Thawed reagents were each vortexed briefly to mix. Adequate Cepheid SmartCap PCR reaction tubes were labeled and placed it in the rack. Using a fresh pipette tip for each tube, 5 ul of the first round PCR master mix were added into each tube. Again with a fresh pipette tip for each specimen, 5 µL of specimen were added to the respective tubes which were then closed without snapping SmartCaps in place. The tubes were centrifuged for 30 seconds in the "SMARTCYCLER" centrifuge and placed in sequence in the

"SMARTCYCLER" instrument with the lid on the "SMARTCYCLER" closed instrument and the run initiated run. The cycling conditions on the Cepheid platform are indicated below (1^{st} round PCR).

Following completion of the run, the tubes were removed and a second round of PCR was set up as follows. The tube lid was opened followed by the addition of 15 ul of the second round PCR master mix into the "SMARTCAP" reservoir to the final volume of 25 ul. The spike was inserted and the lid was snapped into place. The tubes were then centrifuged for 30 seconds in the microcentrifuge with a suitable rotor. The inner PCR reaction was then run for 40 cycles under cycling conditions on the Cepheid platform as indicated below (2nd round PCR). Following completion of the run, the Cepheid tubes were removed and discarded.

The following cycling parameters were used.

| **First Round PCR** | | |
|---|---|---|
| Temperature | Time | Cycles |
| 94C | 2min | 1 |
| 92C | 20sec | |
| 55C | 30sec | 18 |
| 70C | 30sec | |
| 70C | 5min | 1 |

| **Second Round PCR** | | |
|---|---|---|
| Temperature | Time | Cycles |
| 95C | 1min | 1 |
| 95C | 20sec | 40 |
| 59C | 30sec | collection |

The reaction mix for a single quadruplex in the SMARTCAP tubes was prepared using the following individual components.

### • First round PCR (R1)

| Master Mix (MM1) | |
|---|---|
| Reagents | ul |
| DNA template (ul) | 5.00 |
| lOx Magic Buffer | 1 |
| Taq (Ab) Polymerase | 0.5 |
| lOx outer Primer Mix | 1 |
| 2.5 mM dNTPs (100nM) | 0.4 |
| Water | 2.10 |
| Total | 10.0 |
| Outer primer PM-1 final Conc: All /Actin markers-0.05uM -0.04uM | |

### • Second Round PCR (R2)

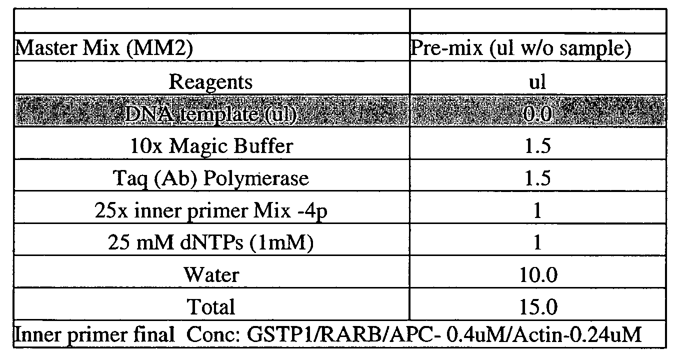

The PCR Master Mixes were prepared as follows (outer primer and inner Scorpion probe/primer mixes)

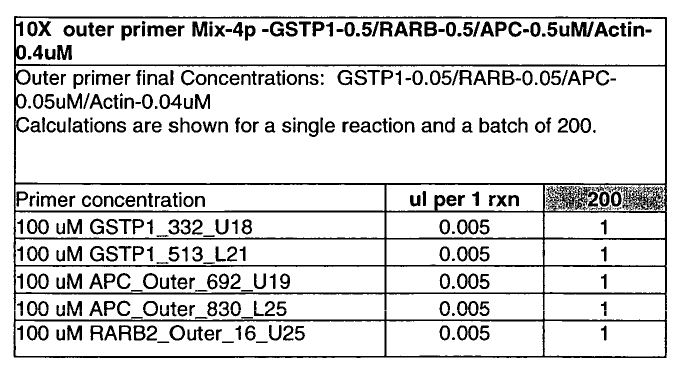

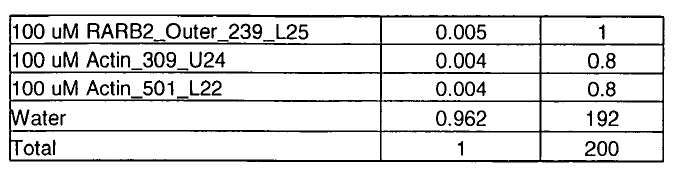

| **25X inner primer/probe Mix -4p (10uM each/6uM actin)** | | |
|---|---|---|
| Inner primer final Concentrations: GSTP1/RARB/APC- 0.4uM/Actin-0.24uM. Calculations are shown for a single reaction and a batch of 200. | | |
| Primer concentration | **ul per 1 rxn** | **200** |
| 100 uM GSTP1_Fam_Sc_1112_L15 | 0.1 | 20 |
| 100 uM GSTPi_1151_L22 | 0.1 | 20 |
| 100 uM RARB2_M_136_AS15_Q570 | 0.1 | 20 |
| 100 uM RARB2_165_L24 | 0.1 | 20 |
| 100 uM APC_M_781_AS15_TR | 0.1 | 20 |
| 100 uM APC_804_L25 | 0.1 | 20 |
| 100 uM Actin_Q670_Sc_382_L15 (Cy5) | 0.06 | 12 |
| 100 uM Actin_425_L27 | 0.06 | 12 |
| Water | 0.28 | 56 |
| Total | 1 | 200 |

The final 25 µl reaction contents were as follows:

| **Component** | **Final Conc in Rxn** |
|---|---|
| (NH₄)₂ SO₄ | 16.6mM |
| Tris pH 8.8 | 67mM |
| MgCl₂ | 6.7mM |
| B-ME | 10mM |
| Each dNTP | 1.25mM |
| Each Primer | 400nM |
| Each Scorpion | 400nM |
| Tris-HCl, pH 8.3 | 4mM |
| KCl | 20mM |
| EDTA | 0.02mM |
| DTT | 0.2mM |
| Nonidet P40 | 0.02% (v/v) |
| Tween 20 | 0.02% (v/v) |
| Glycerol | 2% (v/v) |
| TP6-25 antibody | 0.65 ug |
| Taq Polymerase | 5U |
| Tris-HCI, pH 7.6 | 1.8mM |
| Glycerol | 0.70% |
| ProClin 300 | 0.02% |

Data output from the "SMARTCYCLER" analyzer.

Data were analyzed through the implementation of predetermined thresholds and criteria are shown in Table 1.

**Table 1**

| **Ch #** | **Threshold** | **Valid Min Cycle** | **Valid Max Cycle** | **Bkgd Min Cycle** | **Bkgd Max Cycle** | **Box Car Avg.** |
|---|---|---|---|---|---|---|
| FAM | 30 | 13 | 40 | 5 | 45 | 0 |
| Cy3 | 20 | 13 | 40 | 5 | 45 | 0 |
| TxR | 20 | 13 | 40 | 5 | 45 | 0 |
| Cy5 | 20 | 13 | 40 | 5 | 45 | 0 |

Results were generated and are presented as the following assay performance characteristics: % Sensitivity, Specificity and 95%confidence intervals, calculated for the combination of markers at defined Ct cutoffs for 2 in 1 PCR format. Area under the curve values were calculated based on ROC curve analysis performed with two statistical software packages. For a single marker analysis, AUC values were generated using MedCalc software and for different combinations of multiple markers, logistic regression model in S-Plus statistical software was applied.

A cut-off value was set based on the relative distribution of Ct values between the cancer and non-cancer patients. If either one of Ct values from the set of methylation markers was below the defined cutoff, the sample was considered methylated, even if Actin indicated the "no test" case. The figures show the data compared across a variety of parameters to illustrate the various embodiments of the invention.

### Example 2: Effect of Sample Storage and Panel Identification

The procedure described in example 1 was repeated with combinations assays comprising a combination of Markers from the GSTP1, RARβ2, and APC genes. Assays were conducted within 3 days of sample collection, 5 days of sample collection, and 16 days of sample collection as indicated. Results are shown in Table 2.

**Table 2.**

| **GSTP** | **RARβ2** | **APC** | **Days Stored** | **Sensitivity (%)** | **Specificity (%)** |
|---|---|---|---|---|---|
| X | | | **16** | 31 | 96 |
| | | X | **16** | 31 | 93 |
| | X | | **16** | 36 | 86 |
| X | | X | **16** | 44 | 91 |
| X | X | | **16** | 39 | 85 |
| X | X | X | **16** | 49 | 82 |
| X | | | **5** | 35 | 96 |
| | | X | **5** | 35 | 91 |
| | X | | **5** | 40 | 85 |
| X | | X | **5** | 50 | 90 |
| X | X | | **5** | 44 | 84 |
| X | X | X | **5** | 54 | 81 |
| X | | | **3** | 36 | 91 |
| | | X | **3** | 32 | 88 |
| | X | | **3** | 29 | 93 |
| X | | X | **3** | 54 | 88 |
| X | X | | **3** | 39 | 84 |
| X | X | X | **3** | 54 | 81 |

The sample set for this example were whole (neat) urine samples and consisted of 148 samples (68 known cancers) for the 16 day set, 121 samples (52 known cancers) for the 5 day set, and 73 samples (30 known cancers) for the three day set. Surprisingly, the combination of GSTP and RARβ2 outperformed the combination of GSTP, RARβ2, and APC despite APC being a known prostate cancer marker. This two-gene combination vastly outperformed any other when samples were stored for three days or less. When all tests were considered, the positive predictive value for samples stored for 3 days was 65.9% compared to 51.35 % for samples held for 5 days, and 47.22% for samples stored for 16 days. ROC curves analyses were then conducted for new data sets (N=73, known cancers=30, known non-cancers = 43) using whole urine samples and using sediments prepared as described above and stored for 3 days. The area under the curve was determined for individual Markers. Results are summarized in Table 3

**Table 3**

| **Marker** | **Sample** | **Approximate Sens/Spec (%)** | **AUC** |
|---|---|---|---|
| GSTP | WU | 38/93 | .66 |
| RARβ | WU | 31/93 | .58 |
| APC | WU | 35/98 | .64 |
| GSTP | Sediment | 43/90 | .64 |
| RARβ | Sediment | 56/77 | .64 |
| APC | Sediment | 52/87 | .62 |

GSTP by itself gave the best results by ROC analysis when samples were whole urine (neat). Spinning the sample down to sediments improved the performance of the RARβ Marker tremendously as shown by this same analysis.

### Example 3: Prostate Massage

Two additional samples sets were tested and analyzed to determine the effect of prostatic massage on the performance of the urine based assay. In the first sample set, 36 samples (20 known cancers) were obtained from patients having prostatic massage limited to less than 20 seconds. In the other sample set, 77 samples (30 known cancers) were obtained from patients having prostatic massage for more than 20 seconds. In each case, samples were stored for five days or less. The results of the MSPCR conducted on these samples are summarized in Table 4.

**Table 4**

| **GSTP** | **RARβ2** | **APC** | **Massage (seconds)** | **Sensitivity (%)** | **Specificity (%)** |
|---|---|---|---|---|---|
| X | | | **<20** | 39 | 100 |
| | | X | **<20** | 33 | 93 |
| | | X | **<20** | 39 | 80 |
| | X | | **<20** | 50 | 87 |
| X | | X | **<20** | 56 | 93 |
| X | X | | **<20** | 56 | 87 |
| X | X | X | **<20** | 61 | 87 |
| X | X | X | **<20** | 61 | 93 |
| X | | | **>20** | 33 | 93 |
| | | X | **>20** | 37 | 91 |
| | | X | **>20** | 41 | 76 |
| | X | | **>20** | 37 | 84 . |
| X | | X | **>20** | 48 | 89 |
| X | X | | **>20** | 41 | 82 |
| X | X | X | **>20** | 48 | 82 |
| X | X | X | **>20** | 52 | 82 |

The best results were obtained from the panel that included GSTP, RARβ, and APC when the prostate massage was less than 20 seconds in duration. This is surprising as one would have expected lengthier massage to release more cells and increase, at the least, the specificity.

### Example 4. Digital Rectal Examination

Four additional sample sets were tested and analyzed to determine the effect of selection on the basis of an abnormal versus a normal digital rectal examination (DRE) on the performance of the urine based assay. In the first sample set, 64 whole urine samples (23 known cancers) were obtained from patients having a normal DRE. In the second sample set 33 whole urine samples (19 known cancers) were obtained from patients having an abnormal DRE. The third sample set contained 48 sedimented samples (21 known cancers) who presented with a normal DRE and the fourth sample set contained 22 sedimented samples (8 known cancers) of from patients with abnormal DREs. In each case, samples were stored for five days or less. The results of the MSPCR conducted on these samples are summarized in Table 5.

**Table 5**

| **GSTP** | **RARβ2** | **APC** | **Sample** | **DRE** | **Sens(%)** | **Spec(%)** |
|---|---|---|---|---|---|---|
| X | | | **Whole** | **Negative** | 20 | 95 |
| | | X | **Whole** | **Negative** | 30 | 87 |
| | X | | **Whole** | **Negative** | 25 | 79 |
| X | | X | **Whole** | **Negative** | 35 | 85 |
| X | X | | **Whole** | **Negative** | 25 | 82 |
| X | X | X | **Whole** | **Negative** | 40 | 72 |
| X | X | X | **Whole** | **Negative** | 40 | 74 |
| X | | | **Whole** | **Abnormal** | 60 | 92 |
| | | X | **Whole** | **Abnormal** | 60 | 92 |
| | X | | **Whole** | **Abnormal** | 53 | 85 |
| X | | X | **Whole** | **Abnormal** | 80 | 92 |
| X | X | | **Whole** | **Abnormal** | 67 | 85 |
| X | | | **Sediment** | **Negative** | 19 | 88 |
| | X | | **Sediment** | **Negative** | 10 | 88 |
| | X | | **Sediment** | **Negative** | 48 | 80 |
| | | X | **Sediment** | **Negative** | 33 | 76 |
| X | X | | **Sediment** | **Negative** | 52 | 76 |
| X | | X | **Sediment** | **Negative** | 38 | 72 |
| X | | | **Sediment** | **Abnormal** | 71 | 85 |
| X | | | **Sediment** | **Abnormal** | 86 | 77 |
| | X | | **Sediment** | **Abnormal** | 57 | 62 |
| | X | | **Sediment** | **Abnormal** | 57 | 85 |
| | | X | **Sediment** | **Abnormal** | 86 | 92 |
| X | X | | **Sediment** | **Abnormal** | 71 | 77 |
| X | | X | **Sediment** | **Abnormal** | 86 | 75 |

Markers used with samples selected from patients with an abnormal DRE performed substantially better than the cases in which patients had negative DREs. Whole urine samples from patients with abnormal DREs were assayed with nearly the same degree of sensitivity and specificity as the best sedimented samples when the Marker panel was made up of GSTP and APC. A single Marker (APC) assay performed the best in sedimented samples with an abnormal DRE but the GSTP/APC panel was not far behind.

### Example 5: PSA Level

Two additional samples sets were tested and analyzed to determine the effect of sample selection based on PSA level. In the first sample set, -52 whole urine samples ( 25 known cancers) were obtained from patients having a PSA value of 2.5-4 ng/ml. In the other sample set, 169 samples (80 known cancers) were obtained from patients having a PSA level of 4-10 ng/ml. In each case, samples were stored within five days at 4° C between urine collection and sedimentation procedures. The results of the MSPCR conducted on these samples is summarized in Table 6 below.

For 52 subjects with PSA levels between 2.5 and 4 ng/mL (including 25 cancer and 27 non-cancer cases), sensitivity of 58% and specificity of 88% was demonstrated when using logistic regression model, or 58% and 81%, respectively, when using 3 markers with the following Ct cutoffs: GSTP=26, RAR=28, APC=25 and no test rate at 3.8%. For patient co-hort with PSA 4-10, sensitivity/specificity characteristics were 59/65% using the same markers and Ct cutoffs. Two-sample T-test confirmed that there was no statistically significant difference in assay performance between two co-horts with PSA ranges 2.5-4 and 4-10 ng/mL (P=0.000). Results are shown in Table 6.

**Table 6**

| Marker | GST D2 | RAR | APC | Sens, % | Spec, % | AUC | No test rate, % |
|---|---|---|---|---|---|---|---|
| PSA 2.5-4, n=52, C=25, NC=27 | 28 | | | 38 | 85 | | 3.8 |
| | 26 | | | 26 | 96 | 0.619 | |
| | | 28 | | 54 | 85 | 0.712 | |
| | | | 26 | 25 | 81 | 0.574 | |
| | 26 | | 28 | 54 | 73 | 0.622 | |
| | Logistic regression model | | | 38 | 85 | | |
| | 26 | 28 | | 54 | 81 | 0.697 | |
| | Logistic regression model | | | 58 | 85 | | |
| | 26 | 27 | 25 | 50 | 81 | 0.688 | |
| | 26 | 27 | 25 | 58 | 81 | | |
| | Logistic regression model | | | 58 | 88 | | |
| PSA 4-10, n=169, C=80, NC=89 | 28 | | | 49 | 90 | 0.652 | 5.9 |
| | 26/30 | | | 34/44 | 95/84 | | |
| | | 28/26 | | 48/40 | 70/84 | 0.602 | |
| | | | 26 | 40 | 83 | 0.616 | |
| | 26 | | 28 | 62 | 70 | 0.634 | |
| | 28 | | 27 | 59 | 74 | | |
| | Logistic regression model | | | 45 | 85 | | |
| | 26 | 28 | | 53 | 70 | 0.644 | |
| | Logistic regression model | | | 45 | 85 | | |
| | 26 | 28 | 25 | 59 | 65 | 0.651 | |
| | 26 | 27 | 25 | 56 | 73 | | |
| | Logistic regression model | | | 45 | 87 | | |

### Example 6: More Extensive Multiplexing

More extensive multiplexing was conducted using additional markers known to be useful in prostate cancer analysis. Maximum assay specificity was sought given that the objective of the assay was to resolve ambiguous PSA results (2.5-4 ng/ml in clinical use). The assays were performed on sediment samples stored for 3 days. One round of PCR was conducted on each of 60 samples (30 from cancer cases, 30 from non-cancer samples). Data with urine sediments generated for 6 markers (GST+RAR+APC+RASS+CDH1+PDLIM4) using 3 quadruplex reactions demonstrate that CDH1, RASS and PDLIM4 do not add value to maintain specificity at 80%. Inclusion of all 6 markers impairs assay specificity (sens=44%, spec =63%). Moreover, use of 6 markers does not suit the singletube assay format. Results are shown in Table 7.

**Table 7.**

| GSTP1 | RAR | APC | RASSF1A | CDH1D2 | PDLM4D2 | Sens | Spec |
|---|---|---|---|---|---|---|---|
| X | X | | X | | X | 41 % | 80% |
| X | X | | | X | X | 44% | 70% |
| X | X | | X | | X | 37% | 83% |
| X | X | | | | X | 41 % | 73% |
| X | X | X | X | X | X | 44% | 63% |
| X | X | X | | | | 38% | 80% |

### Example 7: Normalization of Results using S100A2

Urine samples were tested using four markers (GSTP1, RARβ2, APC, and S100A2) in MPCR reactions as described above.

**Table 7.**

| | | | Pru-Mu | | |
|---|---|---|---|---|---|
| | | | GSTP1 | RAR | APC |
| Sensitivity | | | 20.00% | 5.00% | 5.00% |
| Specificity | | | 90.00% | 100.00% | 90.00% |
| | | | GSTP1/APC//RARB2 | | |
| Sensitivity | | | | 20.00% | |
| Specificity | | | | 80.00% | |

The data above show performance of three markers individually and as a combination on a representative set of 20 Cancers and 10 Non-cancers. The sensitivity is lower than typically observed due to the less than optimal storage time of the urine samples prior to sedimentation.

The same samples were then analyzed with S100 and the delta Ct between S100 and the gene of interest was used to generate a cutoff. The data show improved performance for RARβ2 and APC but not GSTP1. This differential effect was observed because the borderline cases for RARβ2 and APC could now be unambiguously assigned as Cancer, improving the sensitivity. There were no borderline cases for GSTP1 in this data set, however, which is why the sensitivity remained unchanged.

**Table 8**

| | Pru-Mu - Norm S100 | | |
|---|---|---|---|
| | GSTP1 | RAR | APC |
| Sensitivity | 20.00% | 15.00% | 20.00% |
| Specificity | 90.00% | 100.00% | 90.00% |
| | | GSTP1/APC//RARB2 | |
| Sensitivity | | 40.00% | |
| Specificity | | 80.00% | |

## Claims

1. A method of detecting prostate cancer comprising, obtaining a urine sample from a person, and determining the methylation status of only the GSTP1 gene and one or more controls in the urine sample no later than three days after said urine sample was obtained; wherein methylation that exceeds a pre-determined value is indicative of prostate cancer and methylation that does not exceed such pre-determined value is indicative of the absence of prostate cancer.

2. A method of detecting prostate cancer comprising, obtaining a urine sample from a person, determining the methylation status of only the S 100 gene and only one more genes selected from the group consisting of the GSTP1 gene, APC, and RARβ2 and one or more controls in the urine sample no later than three days after said urine sample was obtained; wherein if the Ct value of the methylation of the GSTP1, APC, or RARβ2 gene less that of the S 100 gene exceeds a pre-determined value it is indicative of prostate cancer and methylation that does not exceed such pre-determined value is indicative of the absence of prostate cancer.

3. The method according to claim 1 or 2 further comprising measuring the presence of a reference Marker and wherein prior to the collection of said urine sample, the person is subjected to prostatic massage for about 20 seconds.

4. A method of detecting prostate cancer comprising, obtaining a urine sample from a patient, determining the methylation status of only the GSTP1 gene, the RARβ1, the APC gene and one or more controls in the urine sample; wherein methylation that exceeds a pre-determined value is indicative of prostate cancer and methylation that does not exceed such pre-determined value is indicative of the absence of prostate cancer wherein said method is conducted in a single vessel that is left unopened during the conduct of the method.

5. A method of detecting prostate cancer comprising, obtaining a urine sample from a patient with an abnormal DRE, determining the methylation status of only the GSTP1 gene, the RARβ1, the APC gene and one or more controls in the urine sample; wherein methylation that exceeds a pre-determined value is indicative of prostate cancer and methylation that does not exceed such pre-determined value is indicative of the absence of prostate cancer wherein said method is conducted in a single vessel that is left unopened during the conduct of the method.

6. A method of detecting prostate cancer comprising, obtaining a urine sample from a patient with an abnormal DRE, determining the methylation status of only the GSTP1 gene and the RARβ1 and one or more controls in the urine sample; wherein methylation that exceeds a pre-determined value is indicative of prostate cancer and methylation that does not exceed such pre-determined value is indicative of the absence of prostate cancer wherein said method is conducted in a single vessel that is left unopened during the conduct of the method.

7. The method according to claim 4 or 5 or 6 further comprising measuring the presence of a reference Marker.

8. The method of claim 4 or 5 or 6 wherein prior to the collection of said urine sample, the person is subjected to prostatic massage for about 20 seconds.

9. The method of claim 1, 2, 4, 5 or 6 further comprising the steps of determining the PSA level of said person and conducting said method only on people having a PSA level between 2.5 and 4 ng/ml.

10. The method of claim 1, 2, 4, 5 or 6 wherein the methylation status of the genes is determined using nested PCR wherein a first round of PCR is conducted followed by a subsequent round of PCR wherein primers and probes for conducting said subsequent PCR are directed to sequences within the sequences amplified in the first round of PCR and wherein the urine samples are spun down to form sediments prior to conducting the first round of PCR.

11. A kit for conducting an assay to detect prostate cancer, comprising: nucleic acid amplification and detection reagents for detecting the presence of genes consisting essentially of GSTP1 and one or more control genes and instructions that direct its use in patients in having measured PSA levels of 2.5-4 ng/ml.

12. A kit for conducting an assay to detect prostate cancer, comprising: nucleic acid amplification and detection reagents for detecting the presence of genes consisting essentially of GSTP1, RARβ1 and one or more control genes and instructions that direct its use in patients in having measured PSA levels of 2.5-4 ng/ml.

13. A kit for conducting an assay to detect prostate cancer, comprising: nucleic acid amplification and detection reagents for detecting the presence of genes consisting essentially of GSTP, RARβ1, APC and one or more control genes and instructions that direct its use in patients in having measured PSA levels of 2.5-4 ng/ml.

14. A kit for conducting an assay to detect prostate cancer, comprising: nucleic acid amplification and detection reagents for detecting the presence of the S 100A2 gene and one more genes selected from the group consisting of GSTP, RAR β1, and APC, and one or more control genes.
